(19) Europäisches Patentamt / European Patent Office / Office européen des brevets

(11) **EP 4 400 139 B1**

(12) **EUROPEAN PATENT SPECIFICATION**

(45) Date of publication and mention
of the grant of the patent:
**22.04.2026 Bulletin 2026/17**

(21) Application number: **22875200.2**

(22) Date of filing: **30.09.2022**

(51) International Patent Classification (IPC):
***A61M 16/06*** *(2006.01)*

(52) Cooperative Patent Classification (CPC):
(C-Sets available)
**A61M 16/0616; A61M 16/06; A61M 16/0605;**
**A61M 16/0611; A61M 16/0683; A61M 16/0816;**
A61M 16/0633; A61M 2202/0225          (Cont.)

(86) International application number:
**PCT/CN2022/123528**

(87) International publication number:
**WO 2023/051820 (06.04.2023 Gazette 2023/14)**

(54) **GAS DISCHARGE ASSEMBLY AND PATIENT INTERFACE DEVICE**

GASENTLADUNGSANORDNUNG UND PATIENTENSCHNITTSTELLENVORRICHTUNG

ENSEMBLE D'ÉVACUATION DE GAZ ET DISPOSITIF D'INTERFACE PATIENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2021 CN 202111165590**

(43) Date of publication of application:
**17.07.2024 Bulletin 2024/29**

(73) Proprietor: **BMC Medical Co., Ltd.
Shijingshan
Beijing 100041 (CN)**

(72) Inventors:
• **WANG, Yajie
Beijing 100041 (CN)**
• **ZHOU, Mingzhao
Beijing 100041 (CN)**

• **ZHUANG, Zhi
Beijing 100041 (CN)**

(74) Representative: **Grünecker Patent- und
Rechtsanwälte
PartG mbB
Leopoldstraße 4
80802 München (DE)**

(56) References cited:
**WO-A1-2020/136070      WO-A1-2020/208523
WO-A1-2021/046605      CN-A- 104 968 387
CN-A- 105 120 935      CN-A- 105 944 209
CN-A- 113 842 534      CN-U- 217 187 361
US-A- 5 724 965      US-A1- 2015 352 306
US-A1- 2019 030 272      US-A1- 2021 244 905
US-B2- 10 912 909**

**(Cont. next page)**

(52) Cooperative Patent Classification (CPC): (Cont.)

C-Sets
A61M 2202/0225, A61M 2202/0085

**Description**

**TECHNICAL FIELD**

The present disclosure relates to the technical field of treatment of respiratory-related diseases, in particular to an exhaust assembly, a frame assembly, a cushion assembly, and a patient interface device.

**BACKGROUND**

Respiratory-related diseases are accompanied by a series of respiratory disorders characterized by apnea, hypopnea, and hyperpnea. Examples of respiratory diseases include Obstructive Sleep Apnea (OSA), respiratory dysfunction, obesity, and Chronic Obstructive Pulmonary Disease (COPD). The above-mentioned respiratory diseases are usually treated by adopting Continuous Positive Airway Pressure (CPAP) treatment and Non-invasive ventilation (NIV). During treatment, it is unnecessary to insert tubes into a respiratory tract of a patient by means of surgery, but a treatment device (for example a ventilator) is utilized to provide continuous pressure ventilation or variable pressure ventilation to the respiratory tract of the patient through tubes by means of a patient interface device (or referred to as a mask) worn by the patient so as to assist the patient to perform partial or all respiratory actions to maintain a sufficient oxygen level in the body.

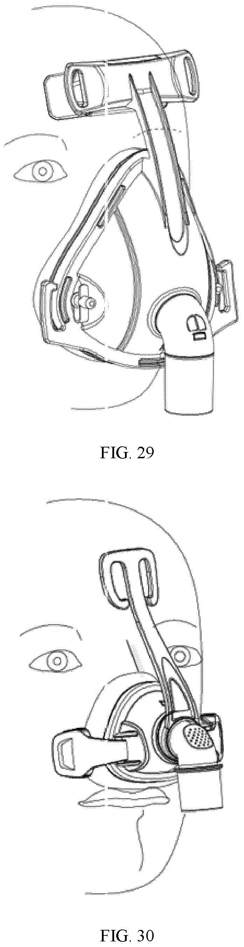

An existing patient interface device (or referred to as a mask) is generally divided into a full-face mask (as shown in FIG. 29), a nasal mask (as shown in FIG. 30), or a nasal pillow mask. The full-face mask has poor comfort, compared with the nasal pillow mask, it has the disadvantages of being not compact and portable, pressing against the nasal bridge, having a poor viewing field, being unfavorable to patients suffering from claustrophobia, and having poor sealing properties for elderly patients suffering from rugged facial features. On the other hand, a nasal pillow of the nasal pillow mask may invade the nostrils, there is an "air jet" effect when air enters the nasal pillow, and therefore, the nasal pillow mask has the disadvantages of intolerance to pressure and nostril stinging and swelling caused by long-term wearing. Therefore, for most patients suffering from Obstructive Sleep Apnea (OSA), the nasal mask is generally used when the condition is not serious or when the mask is worn for treatment for the first time. US20190030272A1 discloses a respiratory mask system comprising a seal, a frame, and a gas delivery conduit, wherein the frame includes a recessed cannel and/or headgear retaining features configured to connect the headgear to the patient interface, and an inlet collar having bias flow holes that connects to a gas delivery conduit. WO2021046605A1 discloses a patient interface system for delivery of respiratory therapy to a patient comprising a nasal portion including a nasal chamber and a nasal sealing portion adapted to form a seal with the patient's face surrounding the entrance to a patient's nares, and the nasal chamber including an opening, an inlet conduit connected to the nasal portion to deliver the pressurized, breathable gas of the nasal chamber, and a nasal chamber coupling component adapted to form part of a releasable ffluid coupling to enable the nasal portion to be fluidly coupled to a mouth portion. US20150352306A1 discloses a patient interface for delivery of a supply of pressurized air or breathable gas to an entrance of a patient's airway comprising a cushion member that includes a retaining structure and a seal-forming structure permanently connected to the retaining structure, a frame ember attachable to the retaining structure, and a positioning and stabilizing structure attachable to the frame member. US20210244905A1 discloses a nasal seal, mask or interface assembly comprising a seal body defining a breathing chamber, wherein a nasal port is provided in the seal body, and the nasal port comprises a central portion straddled by a pair of lateral portions, and the nasal port further includes an upper edge and a lower edge. WO2020208523A1 discloses a patient interface to deliver a flow of air at a positive pressure with respect to ambient air pressure to an entrance to the patient's airways comprising a frame assembly and a cushion assembly configured to removably and repeatably connect to the frame assembly, wherein the frame assembly and the cushion assembly form at least part of a plenum chamber pressurizable to a therapeutic pressure. US10912909B2 discloses a patient interface for delivery of a supply of pressurized air or breathable gas to an entrance of a patient's airways comprising a cushion member that includes a retaining structure and a seal-forming structure permanently connected to the retaining structure, a frame member attachable to the retaining structure, and a positioning and stabilizing structure attachable to the frame member. WO2020136070A1 a patient interface device comprising a frame having a central protion with a patient facing side, an opposite outward facing side, a firs plurality of magnetic elements secured on or in the patient facing side, and a first aperture defined therethrough.

However, a cushion of a traditional nasal mask seals around the apex of the nose of a patient so that the patient feels uncomfortable due to the pressure on his/her nasal bridge. Moreover, there is poor sealing stability between the nasal mask and the nose of the patient, the sealing is easily damaged once the nasal mask is damaged by an external force, for example, when the patient turns over, has face or mouth wiggle or the tube is dragged.

**SUMMARY**

The present disclosure provides an exhaust assembly, a frame assembly, a cushion assembly, and a patient interface device to solve the above-mentioned technical problems.

A first aspect of the present disclosure provides an exhaust assembly disposed on a patient interface device, comprising a main body, wherein the main body includes:

a tapered boss, wherein the tapered boss is provided with first exhaust holes arranged in an array in a circumferential direction of the tapered boss, and the first exhaust holes are formed through kiss off of an upper mold and a lower mold;

the tapered boss includes an inner wall, an outer wall, and an inclined wall disposed between the inner wall and the outer wall, and the first exhaust holes are disposed on the inclined wall.

In an embodiment, an inner aperture of the first exhaust hole is smaller than or greater than an outer aperture of the first exhaust hole.

In an embodiment, an inner aperture of the first exhaust hole is greater than an outer aperture of the first exhaust hole, and a thickness of the inclined wall is 0.7mm to 2.0mm.

In an embodiment, an inner aperture of the first exhaust hole is smaller than an outer aperture of the first exhaust hole, and a thickness of the inclined wall is 0.7mm to 1.5mm.

In an embodiment, the first exhaust hole is in a shape of a long ellipse, a rectangle or a structure composed of at least two relatively parallel straight lines and at least one arc connected to the two straight lines.

In an embodiment, the first exhaust holes are in a divergent arrangement, and at most two first exhaust holes are adjacent to each of the first exhaust holes.

In an embodiment, the first exhaust holes are round holes.

In an embodiment, the first exhaust holes are taper holes.

A second aspect of the present disclosure provides an exhaust assembly, including a main body and first exhaust holes disposed on the main body, the first exhaust holes being disposed to be close to an edge of the main body in a divergent arrangement, and at most two neighboring first exhaust holes being located around each of the first exhaust holes.

In an embodiment, the main body is of an elliptical structure.

In an embodiment, the main body includes a patient side and a patient opposing side, a tapered boss is disposed on the patient side, and the first exhaust holes are disposed on the tapered boss.

A third aspect of the present disclosure provides a frame assembly used for a patient interface device, including:

a frame main body with one side being connected to an intake tube, and the other side being connected to a cushion assembly;

a pair of skeleton arms comprising a first skeleton arm and a second skeleton arm disposed oppositely on the frame main body; and

a connector configured to connect the cushion assembly to the frame main body, wherein the connector and the cushion assembly are sealed to form a closed connection, the connector is provided with a boss raised from an inner side of the frame main body, a recess for accommodating the cushion assembly is formed between the boss and an inner wall of the frame main body, and the boss extends into a bottom of the cushion assembly.

In an embodiment, the frame main body is provided with the exhaust assembly described above.

In an embodiment, the exhaust assembly and the frame main body are integrally molded.

In an embodiment, the skeleton arms are made of PP (Polypropylene).

In an embodiment, the skeleton arms and the frame main body are integrally molded.

A fourth aspect of the present disclosure provides a frame assembly used for a patient interface device, including:

a frame main body with one side being connected to an intake tube, and the other side being connected to a cushion assembly, the cushion assembly is provided with third exhaust holes, and the frame main body is provided with through holes corresponding to the third exhaust holes;

a pair of skeleton arms including a first skeleton arm and a second skeleton arm disposed oppositely on the frame main body; and

a connector configured to connect the cushion assembly to the frame main body, the connector and the cushion assembly are sealed to form a closed connection, the connector is provided with a boss raised from an inner side of the frame main body, a recess for accommodating the cushion assembly is formed between the boss and an inner wall of the frame main body, and the boss extends into a bottom of the cushion assembly.

In an embodiment, the through holes are disposed in pairs on the frame main body, and are symmetrically disposed relative to a longitudinal central line of the frame main body.

A fifth aspect of the present disclosure provides a frame assembly used for a patient interface device, including:

a frame main body with one side being connected to an intake tube, and the other side being connected to a cushion assembly, the intake tube is provided with second exhaust holes;

a pair of skeleton arms including a first skeleton arm and a second skeleton arm disposed oppositely on the frame main body; and

a connector configured to connect the cushion assembly to the frame main body, the connector and the cushion assembly are sealed to form a closed connection, the connector is provided with a boss raised from an inner side of the frame main body, a recess for accommodating the cushion assembly is formed between the boss and an inner wall of the frame main body, and the boss extends into a bottom of the cushion assembly.

In an embodiment, the cushion assembly includes a nasal cushion and the exhaust assembly described above; the nasal cushion is configured to, when being pressed against a basis nasi of a wearer due to a pressurized gas to seal, apply no clamping effect on an ala nasi of a patient.

In an embodiment, the nasal cushion includes an upper part, a lower part opposite to the upper part, and a circumferential side part disposed between the upper part and the lower part, and the upper part and the lower part are surrounded by the circumferential side part to form a cavity; the upper part includes a face contact part to be in contact with a face of a user;

the face contact part includes a middle part as well as a first side part and a second side part located on two sides of the middle part, and the first side part and the second side part are both connected to the circumferential side part; and
the middle part includes a nose opening communicated with the cavity and a periphery sealing part surrounding the nose opening, and the nose opening is configured to surround lower sides of nostrils of a user in response to the nasal cushion being worn by the user, and is adaptively fitted around the nostrils of the user through the periphery sealing part to seal.

A sixth aspect of the present disclosure provides a patient interface device, including:

a cushion assembly including a nasal cushion configured to be in contact with a face of a patient, the nasal cushion is configured to be adaptively fitted around nostrils of the patient in response to the patient interface device being worn by the patient, and the nasal cushion is configured to, when being pressed against the basis nasi of the patient due to a pressurized gas to seal, apply no clamping effect on the ala nasi of the patient;
a frame assembly with two sides being connected to the nasal cushion and a vent tube, respectively; and
a head strap connected to the frame, the head strap is configured to be fixed around the head of the patient in response to the patient interface device being worn by the patient.

In an embodiment, the cushion assembly is the cushion assembly described above.
In an embodiment, the patient interface device further includes the exhaust assembly described above.
In an embodiment, the frame assembly is the frame assembly described above.
In an embodiment, the frame includes a frame main body and a tapered boss disposed on an outer side of the frame main body, the tapered boss is provided with a connecting aperture passing through the tapered boss and the frame main body, and two ends of the connecting aperture are connected to the nasal cushion and the vent tube, respectively.
In an embodiment, the frame includes a frame main body and an elbow rotatably connected to the frame main body, and the elbow is connected to the vent tube in a sealing way.
In an embodiment, the frame is directly connected to an elbow, the elbow is connected to an air delivery tube configured to be connected to a pressure device; and
an inner diameter of the air delivery tube is 15 mm to 22 mm.
In an embodiment, the frame is directly connected to a flexible hose, the flexible hose is connected to an air delivery tube configured to be connected to a pressure device; and an inner diameter of the flexible hose is 12 mm to 15 mm, and the inner diameter of the air delivery tube is 15 mm to 22 mm.
In an embodiment, the flexible hose is an elastic tube.
In an embodiment, the frame is provided with a connector that is rotatably connected to the flexible hose.
In an embodiment, the nasal cushion includes an upper part, a lower part opposite to the upper part, and a circumferential side part disposed between the upper part and the lower part, and the upper part and the lower part are surrounded by the circumferential side part to form a cavity; and the cavity is communicated with the vent tube.
In an embodiment, a connecting body is provided on an inner side of the frame, and the nasal cushion is connected to the frame in a sealing way through the connecting body.
In an embodiment, a base is provided on a lower side of the nasal cushion, and a bottom opening is provided on the base; and the connecting body is configured as a boss raised from the inner side of the frame, a recess is formed between the boss and an inner wall of the frame, the base of the nasal cushion is disposed in the recess, and the boss extends into the bottom opening.
In an embodiment, the patient interface device further includes a first skeleton arm and a second skeleton arm respectively disposed on two ends of the frame, the first skeleton arm and the second skeleton arm extend to upsides of ears along cheeks of the wearer respectively in response to the patient interface device being worn;

the head strap includes a first side head strap connected to the first skeleton arm, a second side head strap connected to the second skeleton arm as well as an upper head strap and a rear head strap that are disposed between the first side head strap and the second side head strap;

the upper head strap and the rear head strap are configured to be fixed respectively around a back and top of the head of the patient in response to the patient interface device being worn by the patient.

Compared with the related art, the advantages of the present disclosure lie in that the nasal cushion is adaptively fitted around the nostrils of a patient, and only covers the nose of the patient, so that the overall patient interface device is compact, light, and not prominent; Moreover, a nasal cushion is adaptively fitted around the nostrils of the patient to seal, thus a stress sensitive part on the nose of the patient will not be pressed. In addition, no nasal pillow is inserted to the nasal cavities of the patient, so that the wearing comfort is further improved; and the nasal cushion is fixed to the head of the patient by a head strap, so that the sealing stability thereof can be further improved, and the sealing is prevented from being affected by an external force.

## BRIEF DESCRIPTION OF THE DRAWINGS

The present disclosure will be described in more detail hereinafter based on embodiments and with reference to accompanying drawings.

FIG. 1 is a schematic perspective view (observed from a front side) of a nasal cushion in embodiment 1 of the present disclosure;
FIG. 2 is a front view (observed from the front side) of the nasal cushion in embodiment 1 of the present disclosure;
FIG. 3 is a top view (observed from an upper side) of the nasal cushion in embodiment 1 of the present disclosure;
FIG. 4 is a bottom view (observed from a lower side) of the nasal cushion in embodiment 1 of the present disclosure;
FIG. 5 is a rear view (observed from a rear side) of the nasal cushion in embodiment 1 of the present disclosure;
FIG. 6 is a schematic perspective view (observed from a left side) of the nasal cushion in embodiment 1 of the present disclosure;
FIG.7 is a sectional view along A-A in FIG. 3;
FIG.8 is a sectional view of along B-B in FIG. 3;
FIG.9 is a sectional view of a nasal cushion in embodiment2 of the present disclosure;
FIG. 10 is a front view (observed from the front side) of a nasal cushion in embodiment 3 and embodiment 4 of the present disclosure;
FIG. 11 is a schematic perspective view (observed from the left side) of the nasal cushion in embodiment 3 and embodiment 4 of the present disclosure;

FIG. 12 is a schematic perspective view (observed from the front side) of a nasal cushion in embodiment 5 of the present disclosure;
FIG. 13 is a schematic perspective view (observed from the left side) of the nasal cushion in embodiment 5 of the present disclosure;
FIG. 14 is a top view (observed from the upper side) of a nasal cushion in embodiment 6 of the present disclosure;
FIG. 15 is a front view (observed from the front side) of the nasal cushion in embodiment 6 of the present disclosure;
FIG. 16 is a schematic perspective view (observed from the front side) of the nasal cushion in embodiment 6 of the present disclosure;
FIG. 17 is a bottom view (observed from the lower side) of the nasal cushion in embodiment 6 of the present disclosure;
FIG. 18 is a bottom view (observed from the lower side) of a nasal cushion in embodiment 7 of the present disclosure;
FIG. 19 is a schematic diagram illustrating a basic structure of a human nose;
FIG. 20a is a schematic perspective view illustrating a patient interface device in an embodiment of the present disclosure, in which the patient interface device is worn by a patient, a first side head strap is buckled, and a second side head strap is unbuckled;
FIG. 20b is a schematic perspective view illustrating a frame in FIG. 20a being connected with a nasal cushion;
FIG. 20c is a schematic view observed from the right side when the patient interface device shown in FIG. 20a is worn by a patient;
FIG. 20d is a schematic view illustrating the nasal cushion being not sealed when the patient interface device shown in FIG. 20a is worn by a patient;
FIG. 20e is a schematic view illustrating the nasal cushion being sealed when the patient interface device shown in FIG. 20a is worn by the patient, where a thick line shows that a periphery sealing part expands and deforms in response to an increase in a cavity pressure, thereby being closely fitted around the nostrils of the patient;
FIG. 21 is a schematic perspective view of a frame of the patient interface device shown in FIG. 20a;
FIG. 22 is a front view of the frame of the patient interface device shown in FIG. 20a;
FIG. 23 is a sectional view of the frame of the patient interface device shown in FIG. 20a;
FIG. 24a is a rear view of the frame of the patient interface device shown in FIG. 20a;
FIG. 24b is a sectional view illustrating the frame shown in FIG. 20a being connected with the nasal cushion;
FIG. 24c is an enlarged view of part I in FIG. 24b;
FIG. 25 and FIG. 26 show different layout ways of first exhaust holes on the frame of the patient interface device shown in FIG. 20a;

FIG. 27a is a schematic perspective view of a patient interface device in another embodiment of the present disclosure;

FIG. 27b and FIG. 27c are schematic structural diagram of a head strap shown in FIG. 27a;

FIG. 28 is a schematic perspective view of a patient interface device in still another embodiment of the present disclosure; and

FIG. 29 and FIG. 30 are schematic diagrams illustrating a patient interface device in the related art being worn.

Reference signs:

100, 200-nasal cushion;

110, 210-upper part; 111a, 211a-face contact part;

120, 220-lower part; 130, 230-front side part; 140, 240-rear side part; 150, 250-cavity;

111, 211-middle part; 112, 212-first side part; 112, 212-second side part;

1111, 2111-nose opening; 1112, 2112-periphery sealing part;

121, 221-base; 122, 222-bottom opening; 123, 223-sealing structure; 124-third exhaust hole;

131-first groove mechanism;

141-upper lip contact part; 142-support part; 143-second groove mechanism;

5-patient interface device;

50-frame; 501-frame main body; 502-tapered boss; 502a-inner wall; 502b-outer wall; 502c-inclined wall; 52-connecting aperture;

51-exhaust part; 511-first exhaust hole;

53-first engaging lug; 54-second engaging lug; 55-connecting body; 56-recess;

60a-first skeleton arm; 60b-second skeleton arm; 61a-first reeving hole; 61b-second reeving hole; 62-first skeleton arm connecting part; 63-second skeleton arm connecting part;

70-head strap; 71a-first side head strap; 71b-second side head strap; 72-upper head strap; 73-rear head strap;

80-vent tube; 81-flexible hose; 82-second connecting end; 83-pipe connector; 84-first connecting end; 90-elbow; and 91-second exhaust hole.

## DETAILED DESCRIPTION OF THE EMBODIMENTS

The present disclosure will be further described below in conjunction with accompanying drawings.

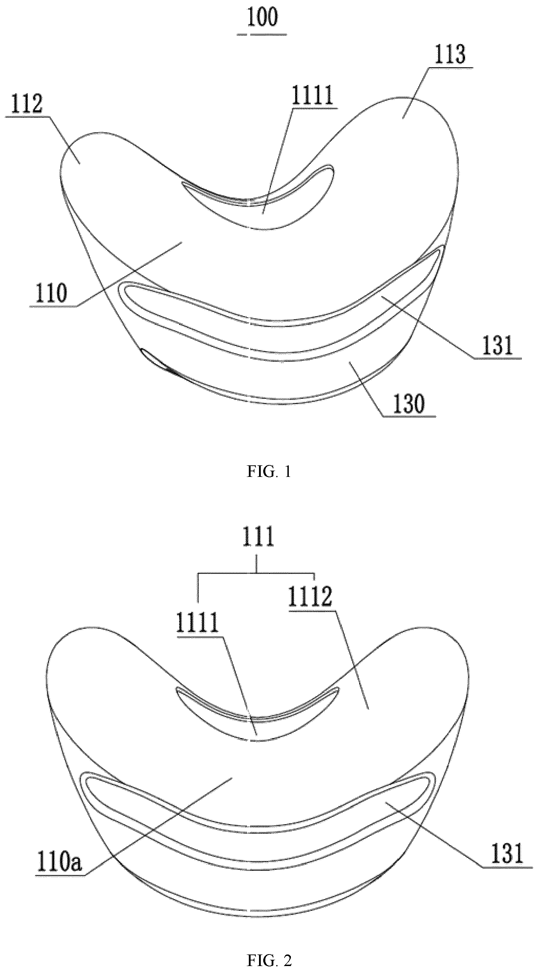

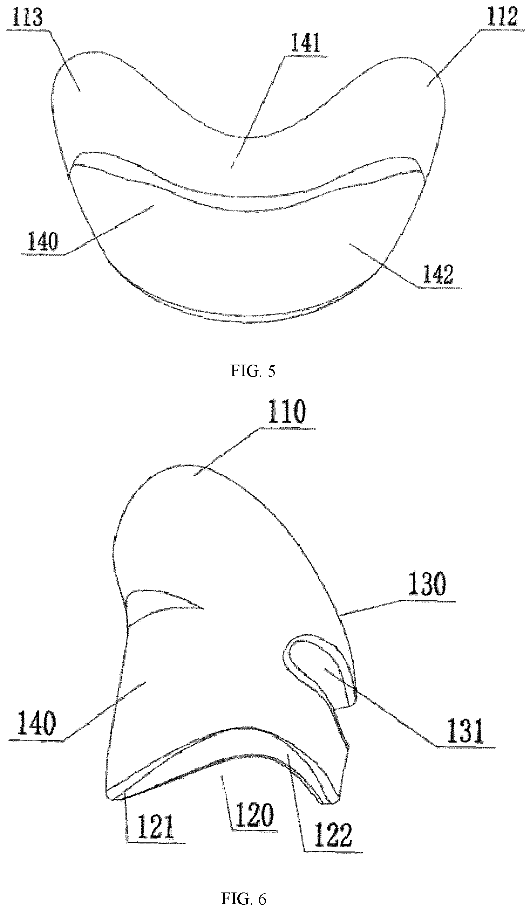

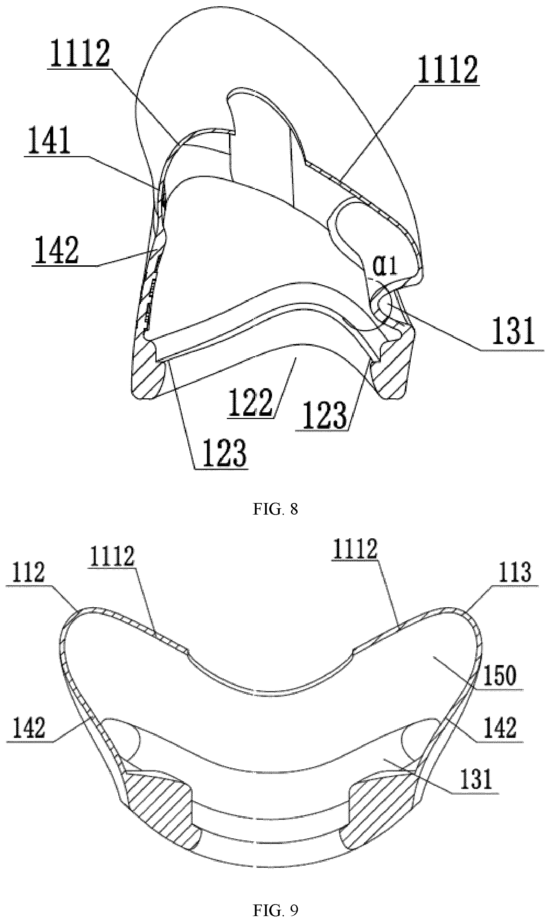

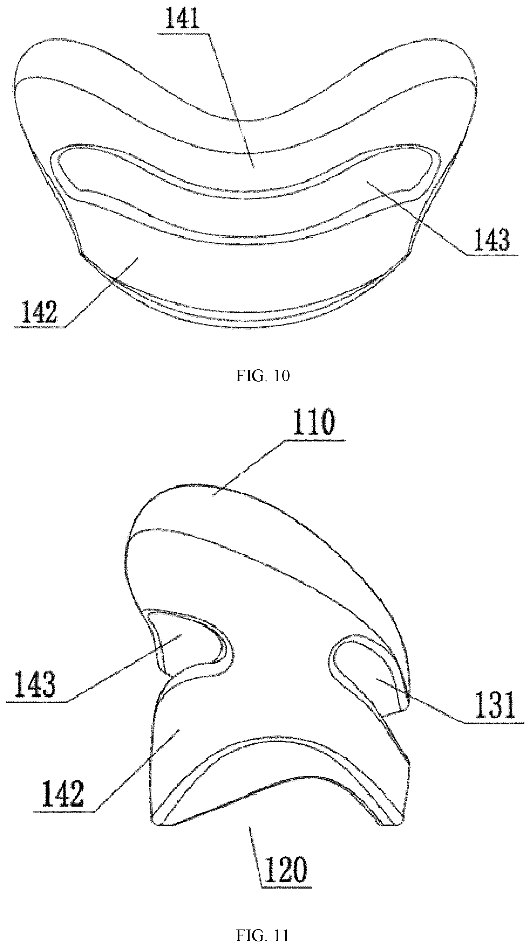

As shown in FIG. 1 to FIG. 28, the present disclosure provides a patient interface device including an exhaust assembly (for example, an exhaust part 51 mentioned hereinafter), a cushion assembly (for example, a nasal cushion 100, 200 mentioned hereinafter), a frame assembly (for example, a frame 50 mentioned hereinafter), a head strap assembly (for example, a head strap 70 mentioned hereinafter), and a vent tube 80.

Two sides of the frame 50 are connected to the nasal cushion 100, 200 and the vent tube 80 respectively in a sealing way, and a pressurized gas may be provided to a patient through the vent tube 80. The head strap 70 is connected to the frame 50, and is configured to be fixed around the head of the patient in response to the patient interface device 5 being worn by the patient. The exhaust part 51 is provided on the frame 50 or the nasal cushion 100, 200, and is configured to enable a side of the patient interface device 5 facing the patient to communicate with the outside, thereby exhausting an airflow exhaled by the patient.

All the assemblies will be described respectively in detail hereinafter.

First, the nasal cushion 100, 200 in the present disclosure will be described in detail. The nasal cushion 100, 200 is used for being in contact with the face of the patient, and is configured to be adaptively fitted around the nostrils of the patient in response to the patient interface device 5 being worn by the patient. Moreover, the nasal cushion 100, 200 is configured to have no clamping effect on two sides of the ala nasi of the patient when the nasal cushion adjoins the basis nasi of a wearer to seal due to a pressurized gas.

As mentioned above, the nasal cushion 100, 200 is configured to be adapted to the frame 50 of the patient interface device 5, and is disposed on the face of the patient, thereby providing the pressurized gas to the patient to perform ventilation treatment. The nasal cushion 100, 200 in the present disclosure will be described below with various specific embodiments.

Embodiment 1

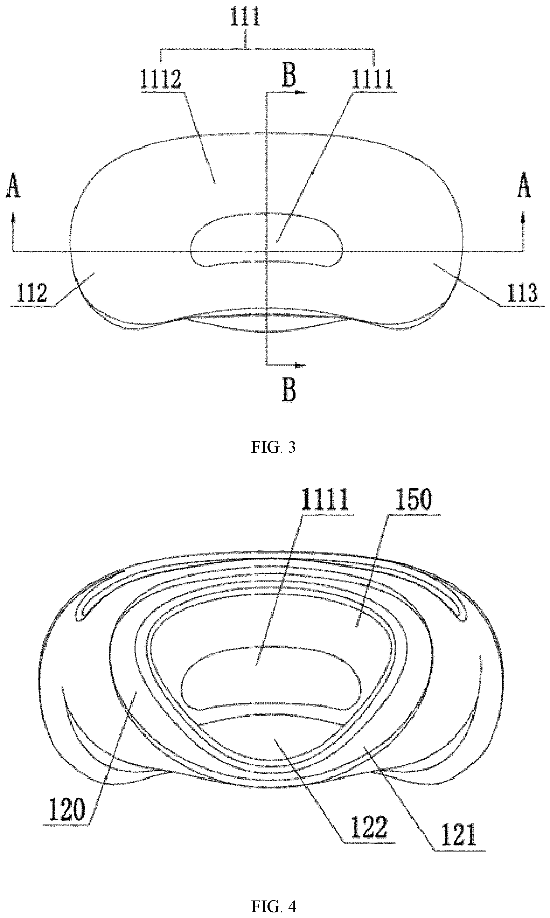

As shown in FIG. 4 to FIG. 11, the nasal cushion 100 in the present embodiment includes an upper part 110, a lower part 120 opposite to the upper part 110, and a circumferential side part disposed between the upper part 110 and the lower part 120. The upper part 110 and the lower part 120 are surrounded by the circumferential side part to form a cavity 150. When a pressurized gas is provided to a patient, the cavity 150 may form an air bag chamber.

The upper part 110 includes a face contact part 111a for being in contact with the face of the patient. Specifically, the face contact part 111a includes a middle part 111 as well as a first side part 112 and a second side part 113 located on two sides of the middle part 111. The first side part 112 and the second side part 113 are both connected to the circumferential side part. It can be understood that, in order to facilitate manufacture, the first side part 112 and the second side part 113 may adopt the same or similar structure, or the first side part 112 and the second side part 113 may be symmetrically disposed relative to the middle part 111.

The middle part 111 includes a nose opening 1111 communicated with the cavity 150 and a periphery sealing part 1112 surrounding the nose opening 1111. The nose opening 1111 is configured to surround lower sides of the nostrils of the patient in response to the nasal cushion 100 being worn by the patient, and is adaptively fitted with the periphery of the nostrils of the patient through the periphery sealing part 1112 so as to seal.

Therefore, the nasal cushion 100 in the present disclosure surrounds the lower sides of the nostrils of the patient, and seals around the nostrils, which can reduce the overall volume of the patient interface device, so that the patient interface device is compact, portable, and not prominent. Moreover, there is no nasal pillow inserted to the patient, the comfort of the patient interface device is further improved.

Further, when the pressurized gas is provided to the patient, there is a certain pressure (for example, the pressure is four to twenty-five hectopascals) in the cavity 150, so that the periphery sealing part 1112 expands and deforms due to the increase of the pressure in the cavity 150, and is in close fit with the periphery of the nostrils of the patient. The nasal cushion 100 in the present disclosure is fitted around the nostrils of the patient due to the expansion and deformation of the periphery sealing part 1112. Therefore, individual differences in noses of patients may not be considered during designing and manufacturing. Accordingly, even if there are individual differences in the noses of the patients, it can also be ensured that the periphery sealing part 1112 is adaptively fitted around the nostrils of the patient due to the deformability of a thin film, thereby improving the sealing properties of contact and the stability of connection between the nasal cushion 100 and the face of the patient.

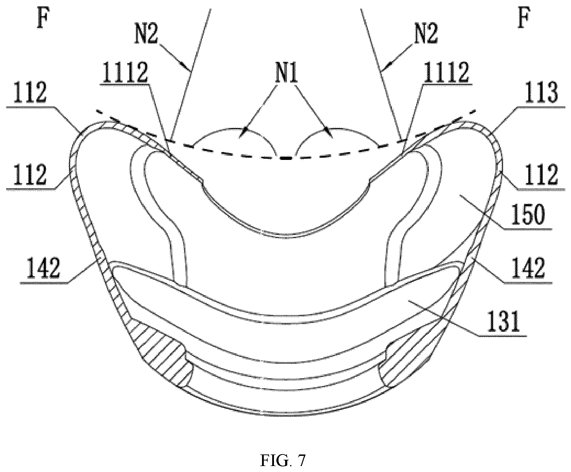

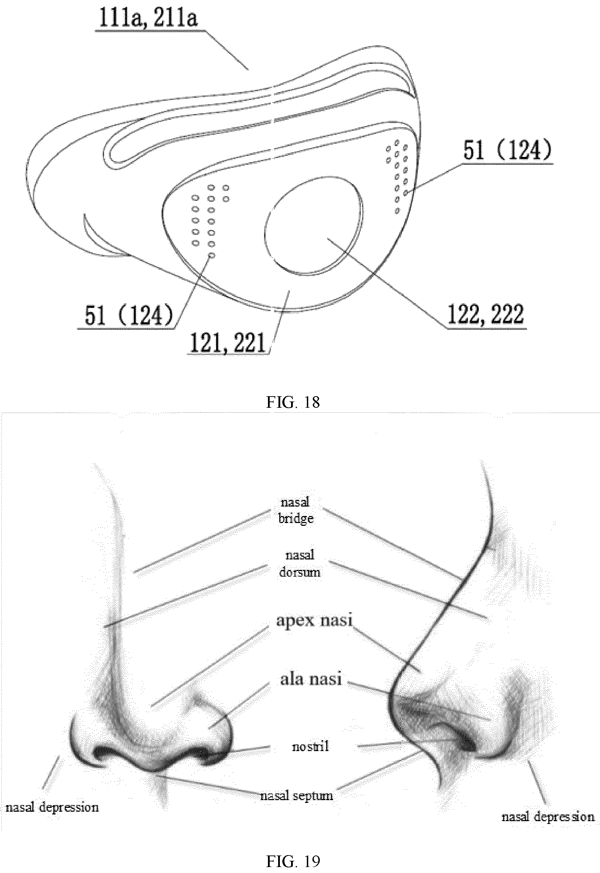

Optionally, the periphery sealing part 1112 is sunken towards the lower part 120, and thus, when the periphery sealing part 1112 expands under the action of the pressure in the cavity 150, the periphery sealing part 1112 will be almost located in the same plane as the first side part 112 and the second side part 113 (a dash line in FIG. 7 indicates a position where the periphery sealing part 1112 is located after the periphery sealing part 1112 expands). Accordingly, the face contact part 111a (the middle part 111) is ensured to horizontally support the bottom of the nose of the patient without wrapping the ala nasi N2 of the patient. Therefore, in the nasal cushion 100 according to the present disclosure, instead of realizing sealing and stable connection by clamping two sides of the ala nasi of the patient, the periphery sealing part 1112 is closely fitted around the nostrils N1 of the patient due to the expansion and deformation of the periphery sealing part 1112. Therefore, the nasal cushion 100 in the present disclosure does not need to be strictly adapted to the width (a distance between the ala nasi on the two sides, as shown in FIG. 19) of the nose of the patient, so that the population to which the nasal cushion 100 can be adapted can be increased, that is, the population adaptability can be improved. In addition, the nasal cushion 100 does not clamp the nose of the patient, which can also reduce the contact area between the nasal cushion and the nose of the patient, thereby further improving the wearing comfort.

Preferably, a distance L between the outer side of the periphery sealing part 1112 and the center of the nose opening 1111 is not greater than a height from the nasal septum to the nasal tip of the patient (as shown in FIG. 19). That is to say, after the nasal cushion 100 is worn by the patient, the outermost edge of the periphery sealing part 1112 will not exceed the nasal tip of the patient, so that the discomfort caused by pressing against the nasal tip of the patient can be avoided either, and the volume thereof can also be reduced.

Optionally, it is further possible that the periphery sealing part 1112 is not sunken, but is located on the same plane as the first side part 112 and the second side part 113, that is, the face contact part 111a may be configured as a planar structure. By such a design, the expansive sealing effect of the periphery sealing part 1112 can be more prominent.

Moreover, the nose opening 1111 may be at least one hole, for example, one, two or more holes, which may be selectively set as required.

The circumferential side part includes a rear side part 140, a front side part 130 opposite to the rear side part 140 as well as a left side part and a right side part that are located between the rear side part 140 and the front side part 130. The rear side part 140, the front side part 130, the left side part and the right side part may be integrally configured.

The rear side part 140 is configured to face the lip of the patient in response to the nasal cushion 100 being worn by the patient, and the rear side part 140 includes an upper lip contact part 141 in contact with the upper lip of the patient and a support part 142 for connecting the upper lip contact part 140 to the lower part 120.

The thickness d1 of the first side part 112, the thickness d2 of the second side part 113, the thickness d3 of the periphery sealing part 1112, the thickness d4 of the support part 142 and the thickness d5 of the upper lip contact part 141 satisfy the following formula:

$$d1=d2, \text{ and } d5=d3 \le d1 \le d4.$$

That is, the thickness of the first side part 112 and the thickness of the second side part 113 are the same, and are both greater than the thickness of the periphery sealing part 1112. For example, the thickness d1 of the first side part 112 and the thickness d2 of the second side part 113 may be both 0.6 to 1.5 mm, preferably, 0.8 to 1.2 mm. The thickness d3 of the periphery sealing part 1112 may be 0.3 to 0.8 mm, preferably, 0.3 to 0.5 mm. Therefore, in the present embodiment, the periphery sealing part 1112 has the minimum thickness, and serves as a thin film region.

When the nasal cushion 100 is worn, the first side part 112 and the second side part 113 are in contact with the face of the patient earlier than the periphery sealing part 1112. Therefore, the first side part 112 and the second side part 113 are important supporting points for the face when the nasal cushion is worn, and the stress on the first side part 112 and the second side part 113 is greater than the stress on the periphery sealing part 1112. Moreover, the first side part 112 and the second side part 113 are located on two sides of the ala nasi of the patient, and are closer to muscles of the cheeks of the patient than the periphery sealing part 1112, so that the patient does not significantly feel the stresses on corresponding positions of the first side part 112 and the second side part 113. Accordingly, the thickness of the first side part 112 and the thickness of the second side part 113 are set to be greater than the thickness of the periphery sealing part 1112, so that the rigidity of both the first side part 112 and the second side part 113 is greater than the rigidity of the periphery sealing part 1112, thereby effectively distributing a pressure generated when the nasal cushion 100 is worn on the face, and improving the wearing comfort.

The support part 142 may extend in the circumferential direction to form for example a part of the rear side part 140, the left side part and the right side part. Therefore, the support part 142 needs to keep the overall shape of the cavity 150, so that the shape of the cavity is kept without collapse when the patient interface device is worn at low pressure and the nasal cushion 100 is pressurized. Therefore, the support part 142 has the maximum thickness d4, which may be, for example, 0.9 to 2.0 mm, preferably 0.9 to 1.5 mm, to ensure certain rigidity.

Since the upper lip contact part 141 corresponds to a stress sensitive part on the face of the patient, the thickness d5 of the upper lip contact part 141 may be the same as the thickness d3 of the periphery sealing part 1112. Therefore, the upper lip contact part 141 in the present embodiment is also a thin film region, so as to ensure that the stress on the upper lip of the patient is small enough when it is worn, thereby improving the wearing comfort.

The front side part 130 and the rear side part 140 each are provided with a groove mechanism, or neither the front side part 130 nor the rear side part 140 is provided with a groove mechanism, or one of front side part 130 and the rear side part 140 is provided with a groove mechanism.

For example, in the present embodiment, the front side part 130 is provided with a first groove mechanism 131 sunken towards the rear side part 140. The first groove mechanism 131 is configured as a groove, an included angle α1 which is an acute angle (for example, 20° to 70°) is formed by two opposite inner walls of the groove, and the thickness of the first groove mechanism 131 may be 0.5 to 1.4 mm. Therefore, when the periphery sealing part 1112 cannot be effectively fitted to the nose of the patient due to a slight pressurized expansion effect under the action of a low pressure, the first groove mechanism 131 can provide certain elastic support at a position near the apex of the nose of the patient, thereby increasing the sealing force. At the same time, different nasolabial angles of different persons can be compressively adapted by compressing the first groove mechanism 131 (that is, by reducing the included angle α1), a more appropriate wearing state can be self-adaptively adjusted.

The lower part 120 includes a base 121 in which a bottom opening 122 communicated with the cavity is provided. The bottom opening 122 is provided with a sealing structure for connecting the base 121 to the frame of the patient interface device in a sealing way.

The bottom opening 122 may be triangular, round or elliptic, etc. The sealing structure in the base 121 is used to seal the base 121 to the frame of the patient interface device, so that the base 121 is connected to the frame of the patient interface device in a nonrotatable way. The sealing structure may be a bulge on an inner wall of the bottom opening 122, and is in interference fit with the frame to realize sealing.

Optionally, the sealing structure may also be a plastic part or silicon rubber with higher hardness, which realizes sealing connection with the frame through the elastic deformation thereof.

The nasal cushion 100 is preferably made of silicon rubber, and may also be made of a material such as foam, thermoplastic elastomer, resin or textiles.

Embodiment 2

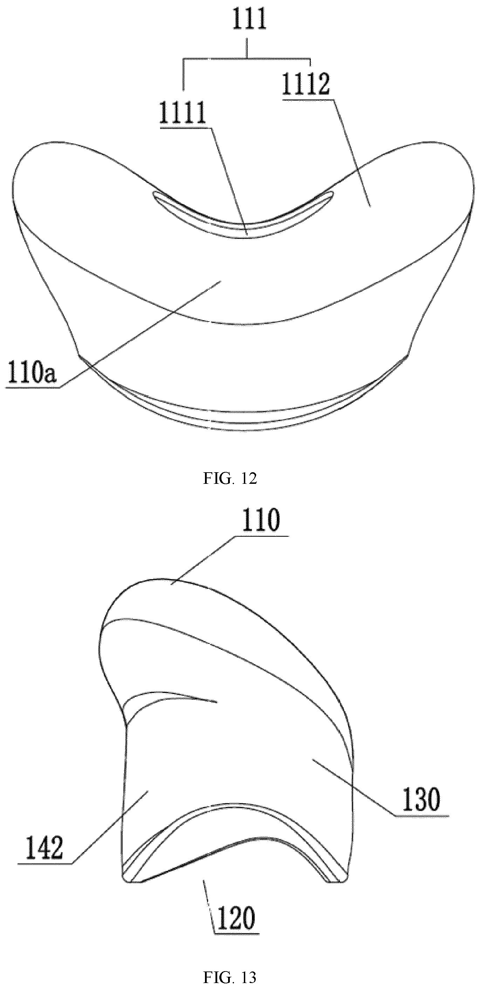

On the basis of embodiment 1, the present disclosure also provides a variant embodiment, i.e., embodiment 2, as shown in FIG. 12. Differences from embodiment 1 will be mainly described as below.

The present embodiment differs from embodiment 1 in that the thickness d1 of the first side part 112 is the same as the thickness d2 of the second side part 113, and is equal to the thickness d3 of the periphery sealing part 1112, that is, d1=d2=d3. The thickness of the first side part 112, the thickness of the second side part 113 and the thickness of the periphery sealing part 1112 may all be 0.3 to 0.8 mm. In this way, the overall face contact part 111a may form an air bag as an integer, so that the stress on the overall face contact part 111a is reduced, and then, the wearing comfort is enhanced. Therefore, the nasal cushion 100 in the present embodiment can be better fitted to facial features of some persons suffering from facial deformity.

In the present embodiment, it is similar to embodiment 1 that the support part 142 may still adopt the greater thickness d4, for example, d4≥d3. For example, d4 may be0.9 to 2.0 mm, preferably 0.9 to 1.5 mm, to ensure certain rigidity.

The features and structures, which are the same as those in embodiment 1, in the present embodiment will not be no longer repeated.

Embodiment 3

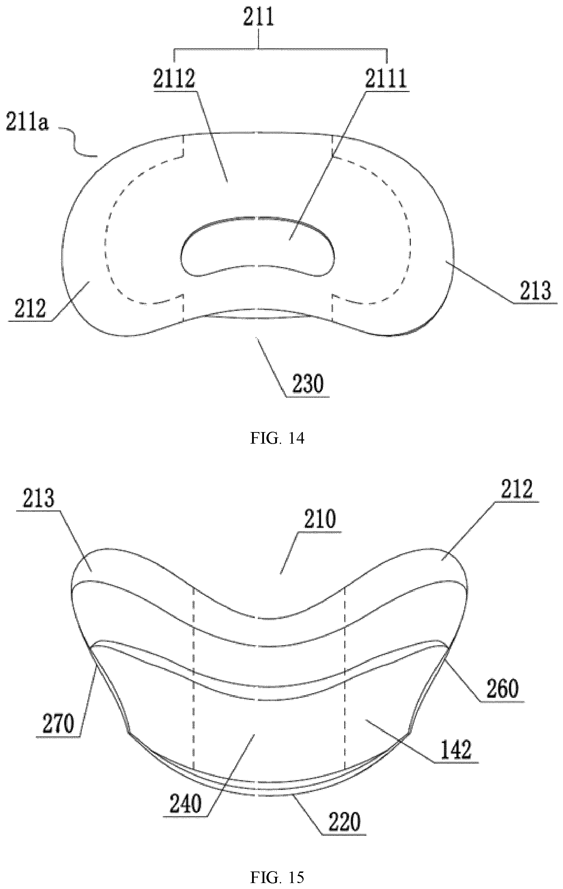

On the basis of embodiment 1, the present disclosure also provides a variant embodiment, i.e., embodiment 3, as shown in FIG. 13 and FIG. 14. Differences from embodiment 1 will be mainly described as below.

The present embodiment differs from embodiment 1 in that the support part 142 is provided with a second groove mechanism 143 sunken towards the front side part 130 in addition to providing the first groove mechanism 131 sunken towards the rear side part 140 on the front side part 130. The second groove mechanism 143 may also be configured as a groove, an included angle $\alpha2$ which is an acute angle (for example, 20° to 70°) is formed by two opposite inner walls of the groove, and the thickness of the second groove mechanism 143 may be 0.5 to 1.4 mm. When the periphery sealing part 1112 cannot be effectively fitted to the nose of the patient due to a slight pressurized expansion effect under the action of a low pressure, the groove can provide certain elastic support at a position near the upper lip of the patient, thereby increasing the sealing force. Moreover, different nasolabial angles of different persons can be compressively adapted by compressing the first groove mechanism 131 (that is, reducing the included angle $\alpha1$) and the second groove 143 (that is, reducing the included angle $\alpha2$), a more appropriate wearing state can be self-adaptively adjusted.

Embodiment 4

On the basis of embodiment 2, the present disclosure also provides a variant embodiment, i.e., embodiment 4, as shown in FIG. 13 and FIG. 14. Differences from embodiment 2 will be mainly described as below.

The present embodiment differs from embodiment 2 in that the support part 142 is provided with a second groove mechanism 143 sunken towards the front side part 130 in addition to providing the first groove mechanism 131 sunken towards the rear side part 140 on the front side part 130. The second groove mechanism 143 may also be configured as a groove, an included angle $\alpha2$ which is an acute angle (for example, 20° to 70°) is formed by two opposite inner walls of the groove, and the thickness of the second groove mechanism 143 may be 0.5 to 1.4 mm. When the periphery sealing part 1112 cannot be effectively fitted to the nose of the patient due to a slight pressurized expansion effect under the action of a low pressure, the groove can provide certain elastic support at a position near the upper lip of the patient, thereby increasing the sealing force. Moreover, different nasolabial angles of different persons can be compressively adapted by compressing the first groove mechanism 131 (that is, reducing the included angle $\alpha1$) and the second groove 143 (that is, reducing the included angle $\alpha2$), a more appropriate wearing state can be self-adaptively adjusted.

Embodiment 5

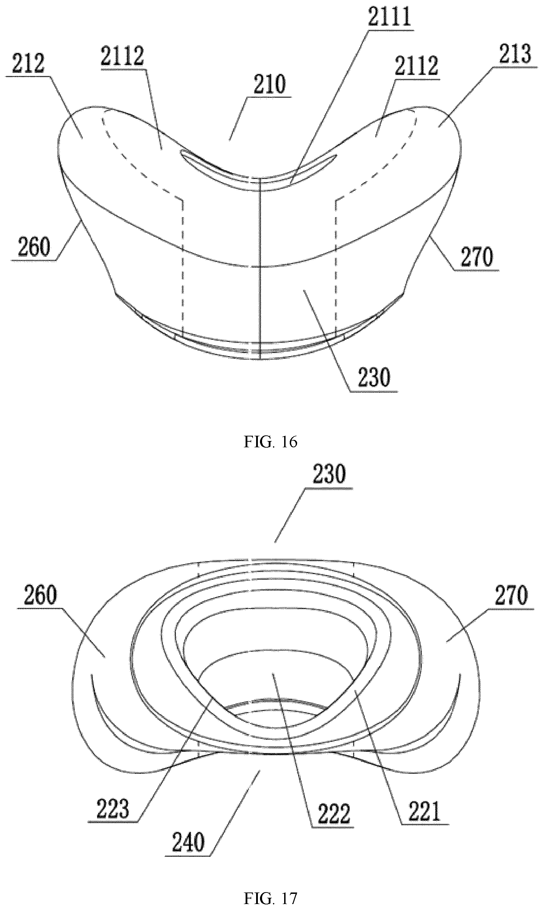

On the basis of embodiments 1, 2, the present disclosure also provides a variant embodiment, i.e., embodiment 5, as shown in FIG. 15 and FIG. 16. Differences from embodiments 1, 2 will be mainly described as below.

The present embodiment differs from embodiments 1, 2 in that the first groove mechanism 131 on the front side part 130 is omitted. That is, in the present embodiment, neither the front side part 130 nor the rear side part 140 is provided with a groove mechanism. Optionally, the thickness of the front side part 130 may be 0.3 to 0.8 mm, that is, the front side part 130 and the periphery sealing part 1112 have the same thickness, and are both thin film regions.

Optionally, the thickness of a part of the front side part 130 is the same as the thickness of the periphery sealing part 1112, for example, the thickness may be 0.3 to 0.8 mm. The thickness of another part of the front side part 130 is the same as the thickness of the support part 142, for example, the thickness may be 0.9 to 2.0 mm, preferably 0.9 to 1.5 mm, to ensure certain rigidity.

Embodiment 6

As shown in FIG. 17 to FIG. 20, the nasal cushion 200 in the present embodiment includes an upper part 210, a lower part 220 opposite to the upper part 210, and a circumferential side part disposed between the upper part 210 and the lower part 220. The upper part 210 and the lower part 220 are surrounded by the circumferential side part to form a cavity, and when a pressurized gas is provided to a patient, the cavity 250 may form an air bag chamber. In addition, the upper part 210 includes a face contact part 211a to be contact with the face of the patient.

The face contact part 211a includes a middle part 211 as well as a first side part 212 and a second side part 213 located on two sides of the middle part 211. The first side part 212 and the second side part 213 are both connected to the circumferential side part. It can be understood that, in order to facilitate manufacture, the first side part 212 and the second side part 213 may adopt the same or similar structure, or the first side part 212 and the second side part 213 may be symmetrically disposed relative to the middle part 211.

The middle part 211 includes a nose opening 2111 communicated with the cavity 250 and a periphery sealing part 2112 surrounding the nose opening 2111. The nose opening 2111 is configured to surround lower sides of the nostrils of the patient in response to the nasal cushion 200 being worn by the patient, and is adaptively fitted around the nostrils of the patient through the periphery sealing part 2112 so as to seal.

Therefore, the nasal cushion 200 in the present disclosure surrounds the lower sides of the nostrils of the patient to seal, which can reduce the whole volume of the patient interface device, so that the patient interface device is compact, portable, and not prominent. Moreover, there is no nasal pillow inserted to the patient, the comfort of the patient interface device is further improved.

Further, when the pressurized gas is provided to the patient, there is a certain pressure (for example, the pressure is four to twenty-five hectopascals) in the cavity 250, so that the periphery sealing part 2112 expands and deforms due to the increase of the pressure in the cavity 250, and is in close fit with the periphery of the nostrils of the patient. The nasal cushion 200 in the present disclosure is fitted around the nostrils of the patient due to the expansion and deformation of the periphery sealing part 2112. Therefore, individual differences in noses of patients may not be considered during designing and manufacturing. Accordingly, even if there are individual differences in the noses of the patients, it can also be ensured that the periphery sealing part 2112 is adaptively fitted around the nostrils of the patient due to the deformability of a thin film, thereby improving the sealing properties of contact and the stability of connection between the nasal cushion 200 and the face of the patient.

Optionally, the periphery sealing part 2112 is sunken towards the lower part 220, and thus, when the periphery sealing part 2112 expands under the action of the pressure in the cavity 250, the periphery sealing part 2112 will be almost located in the same plane as the first side part 212 and the second side part 213 (a dash line in figure indicates a position where the periphery sealing part 2112 is located after the periphery sealing part 2112 expands). Accordingly, the face contact part 211a (the middle part 211) is ensured to horizontally support the bottom of the nose of the patient without wrapping the ala nasi of the patient. Therefore, in the nasal cushion 200 according to the present disclosure, instead of realizing sealing and stable connection by clamping two sides of the ala nasi of the patient, the periphery sealing part 2112 is closely fitted around the nostrils of the patient due to the expansion and deformation of the periphery sealing part 2112. Therefore, the nasal cushion 200 in the present disclosure does not need to be strictly adapted to the width (a distance W between the ala nasi on the two sides) of the nose of the patient, so that the population to which the nasal cushion 200 can be adapted can be increased, that is, the population adaptability can be improved. In addition, the nasal cushion 200 does not clamp the nose of the patient, which can also reduce the contact area between the nasal cushion and the nose of the patient, thereby further improving the wearing comfort.

Preferably, a distance L between the outer side of the periphery sealing part 2112 and the center of the nose opening 2111 is not greater than a height from the nasal septum to the nasal tip of the patient. That is to say, after the nasal cushion 200 is worn by the patient, the outermost edge of the periphery sealing part 2112 will not exceed the nasal tip of the patient, so that the discomfort caused by pressing against the nasal tip of the patient can be avoided either, and the volume thereof can also be reduced.

Optionally, it is further possible that the periphery sealing part 2112 is not sunken, but is located on the same plane as the first side part 212 and the second side part 213, that is, the face contact part 211a may be configured as a planar structure. By such a design, the expansive sealing effect of the periphery sealing part 1112 can be more prominent.

Moreover, the nose opening 2111 may be at least one hole, for example, one, two or more holes, which may be selectively set as required.

The circumferential side part includes a rear side part 240, a front side part 20 opposite to the rear side part 240 as well as a left side part 260 and a right side part 270 that are located between the rear side part 240 and the front side part 230. Main differences of the present embodiment from embodiments 1 to 5 lie in that the periphery sealing part 2112 in the present embodiment respectively extends forward to the front side part 230 and extends backward to the rear side part 240, and the joints between the periphery sealing part 2112 and each of the front side part 230 and the rear side part 240 are continuous curved surfaces, or the joints may be staggered with each other.

Preferably, the periphery sealing part 2112 extends forward to the front side part 230 and extends backward to the rear side part 240, thereby forming an entirety, i.e., the middle part, together with the front side part 230 and the rear side part 240, so that the middle part has a uniform thickness. That is, the thickness d3 of the periphery sealing part 2112 is the same as the thickness of the front side part 230 and the thickness of the rear side part 240. For example, d3 may be 0.3 to 0.9 mm, preferably 0.3 to 0.5 mm, that is, the middle part is a thin film region (a region indicated by a dash line in the figure).

It can be understood that the thickness d5 of the upper lip contact part corresponding to the rear side part 240 and the thickness d4 of the support part are the same, and are both the same as the thickness d3 of the periphery sealing part 2112, that is, d3=d4=d5.

Further, the first side part 212 extends to the left side part 260, the second side part 213 extends to the right side part 270, and a joint between the first side part 212 and the left side part 260 and a joint between the second side part 213 and the right side part 270 are continuous curved surfaces, or the joints may be staggered with each other.

Preferably, the first side part 212 extends to form an entirety together with the left side part 260, the second side part 213 extends to form an entirety together with the right side part 270, that is, a peripheral part, and the peripheral part has a uniform thickness. Therefore, the thickness d1 of the first side part 212, the thickness of the left side part 260, the thickness d2 of the second side part 213 and the thickness of the right side part 270 are all the same. Accordingly, the entirety formed also has a uniform thickness greater than the thickness d3 of the periphery sealing part 2112, that is, d1≥d3. For example, d1 and d2 may be both 0.6 to 1.5 mm, preferably 0.8 to 1.2 mm.

That is to say, in the present embodiment, the rigidity of the peripheral part (the left side part 260, the right side part 270, the first side part 212, and the second side part 213) is greater than the rigidity of the middle part (the periphery sealing part 2112, the front side part 230, and the rear side part 240). The reason is that the peripheral part is a major supporting point for the face of the patient when the nasal cushion 200 is worn, and the stress on the peripheral part is greater than the stress on the middle part corresponding to the nose of the patient. Moreover, the peripheral part is closer to the muscles of the cheeks of the patient, the patient does not significantly feel the stress. Therefore, the rigidity of the peripheral part is set to be greater than the rigidity of the middle part, so that a pressure generated when the cushion is worn on the face is effectively distributed, and the wearing comfort is improved.

The lower part 220 includes a base 221 in which a bottom opening 222 communicated with the cavity is provided. The bottom opening 222 is provided with a sealing structure for connecting the nasal cushion 200 to the frame of the patient interface device in a sealing way.

The bottom opening 222 may be triangular, round or elliptic, etc. The sealing structure in the base 221 is used to seal the base 221 to the frame of the patient interface device, so that the base 221 is connected to the frame of the patient interface device in a nonrotatable way. The sealing structure may be a bulge on an inner wall of the bottom opening 222, and is in interference fit with the frame to realize sealing.

Optionally, the sealing structure may also be a plastic part or silicon rubber with higher hardness, and realizes sealing connection with the frame by means of elastic deformation thereof.

The nasal cushion 200 is preferably made of silicon rubber, and may also be made of a material such as foam, thermoplastic elastomer, resin or textiles.

Embodiment 7

On the basis of various embodiments described above, the present disclosure also provides a variant embodiment, i.e., embodiment 7, as shown in FIG. 18. The present embodiment differs from all of the forementioned embodiments in that an exhaust part 51, i.e., a third exhaust part, is provided on the base 121, 221.

The third exhaust part may be third exhaust holes 124 disposed on two sides of the bottom opening 122. The arrangement and size of the third exhaust holes 124 may be set as required, which are not limited herein.

It should be noted that, in the present embodiment, the exhaust part 51 is provided on the nasal cushion 100, 200. However, in embodiments 1 to 6, the nasal cushion 100, 200 is not provided the exhaust part 51, and the exhaust part 51 is provided on the frame 50, as mentioned hereinafter.

The frame 50 and the exhaust part 51 in the present disclosure will be described in detail as below. It can be understood that the frame 50 and the exhaust part 51 described below may be combined with any one of the nasal cushions 100, 200 in embodiments 1 to 7.

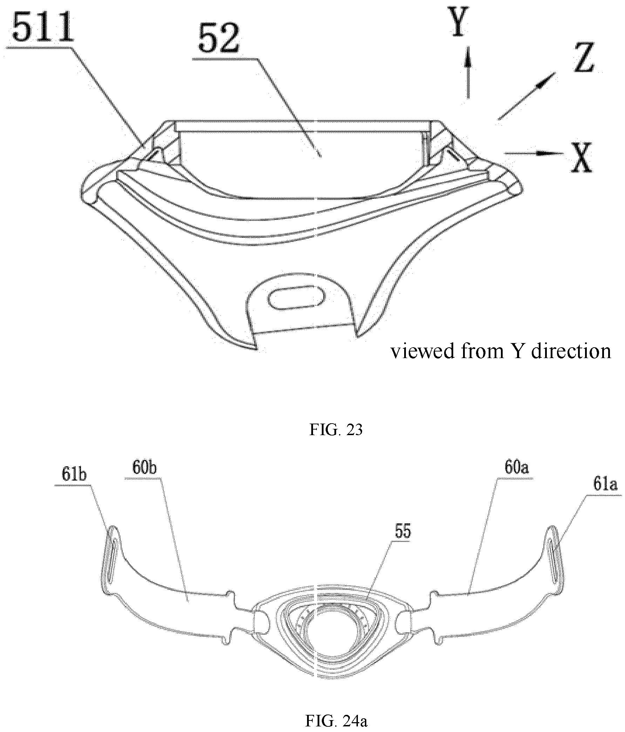

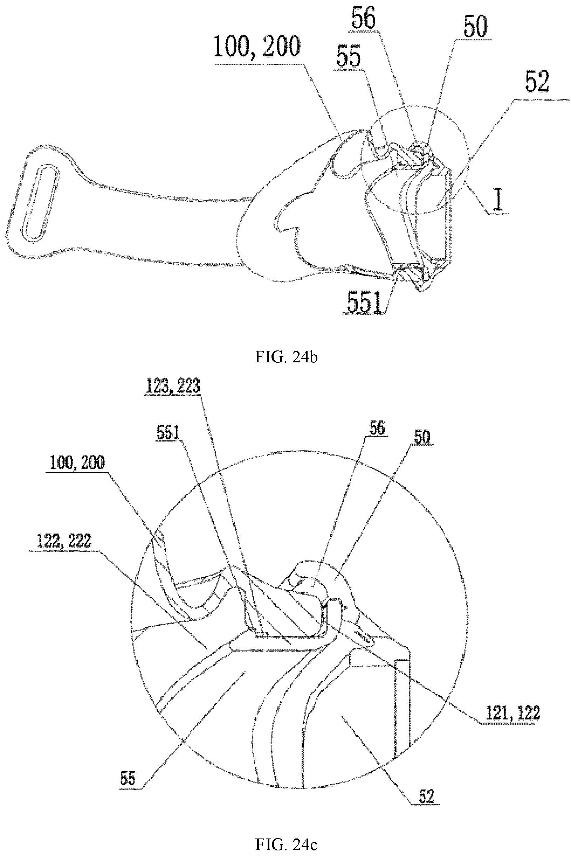

For example, with reference to FIG. 24a, a connecting body 55 may be disposed on the inner side of the frame 50, and a tight coupling between the nasal cushion 100, 200 and the frame 50 is realized by the connecting body 55. The connecting body 55 forms closed connection with the nasal cushion 100, 200 by sealing. The connecting body 55 may be integrated with the frame 50 by means of gluing, buckling and the like. Specifically, the connecting body 55 is configured as a boss raised from the inner side of the frame 50, a recess 56 is formed between the boss and an inner wall of the frame 50, the base 121, 122 of the nasal cushions 100, 200 is disposed in the recess 56, and the boss extends into the bottom opening 122, 222.

Further, with reference to FIG. 24b, FIG. 24c, FIG. 8, and FIG. 17, the outer wall of the connecting body 55 is provided with a sealing ring 551 that is matched with the sealing structure 123, 223 in the bottom opening 122, 222, so that the tight coupling between the nasal cushion 100, 200 and the frame 50 is realized.

It can be understood that the connecting body 55 may be configured as a structure adapted to the bottom opening 122, 222 in shape.

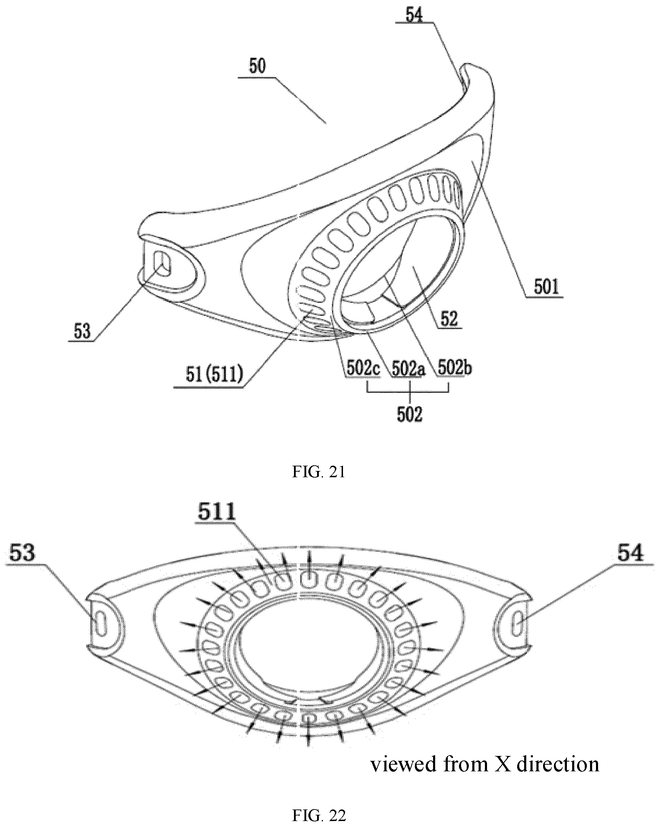

In a preferred implementation, as shown in FIG. 21, the frame 50 includes a frame main body 501 and a tapered boss 502 disposed on the outer side of the frame main body 501. The tapered boss 502 is configured to be gradually reduced in a direction away from the frame main body 501, that is to say, an end, away from the frame main body 501, of the tapered boss 502 is smaller than an end close to the frame main body 501, so that a side wall of the tapered boss 502 forms an inclined wall. Optionally, the tapered boss 502 is configured to be of a solid structure.

Further, as shown in FIG. 21, optionally, the tapered boss 502 includes an inner wall 502a, an outer wall 502b, and an inclined wall 502c disposed between the inner wall 502a and the outer wall 502b. In the optional implementation, the thickness of the inclined wall 502c is 0.7 mm to 2.0 mm, preferably 0.7 mm to 1.5 mm. It should be noted that the inclined wall 502c may have a uniform or nonuniform thickness. When the inclined wall 502c has the nonuniform thickness, the above-mentioned thickness of the inclined wall 502c refers to an average thickness of the inclined wall 502c, which is 0.7 mm to 2.0 mm. Preferably, the average thickness of the inclined wall 502c is 0.7 mm to 1.5 mm.

It can also be understood that a distance between the inner wall 502a and the outer wall 502b is 0.7 mm to 2.0 mm, preferably 0.7 mm to 1.5 mm. It can be understood that, when the inner wall 502a and the outer wall 502b are not parallel with each other, the distance between the inner wall 502a and the outer wall 502b refers to an average distance between the inner wall 502a and the outer wall 502b.

Since the frame 50 is worn on the face of the patient, the frame 50 may include a patient side and a patient opposing side, and the tapered boss 502 is disposed on the patient side.

Further, the tapered boss 502 is provided with a through hole (connecting aperture 52) passing through the tapered boss 502 and the frame main body 501, the connecting aperture 52 communicates with the nasal cushion 100, 200 and a vent tube 80 in a sealing way, respectively. The connecting aperture 52 may be disposed at a central position of the tapered boss 502, and is symmetrically disposed relative to a longitudinal central line of the frame main body 501.

With reference to implementations in FIG. 21 to FIG. 23, the exhaust assembly and the frame 50 are integrally molded. Therefore, it can be understood that the exhaust assembly includes a main body and an exhaust part 51, the main body includes the above-mentioned tapered boss 502, and the exhaust part 51 is disposed on the tapered boss 502. The exhaust part 51 includes a first exhaust part disposed on the inclined wall 502c of the tapered boss 502, and the first exhaust part includes a plurality of first exhaust holes 511 disposed in the circumferential direction of the connecting aperture 52. Since the first exhaust holes 511 are disposed on the inclined wall of the tapered boss 502, when observed from the radial direction of the tapered boss 502, airflows exhausted from the first exhaust holes 511 diverge in the radial direction (X direction) of the tapered boss 502; and when observed from the axial direction (Y direction) of the tapered boss 502, airflows exhausted from the first exhaust holes 511 diverge in the axial direction (Y direction) of the tapered boss 502, a resultant force direction of the airflows exhausted from the first exhaust holes 511 is a direction (Z direction) perpendicular to the axial direction of the tapered boss 502 and the radial direction of the tapered boss 502.

In some optional implementations, an inner aperture of the first exhaust hole 511 is smaller than an outer aperture thereof; in these embodiments, the thickness of the inclined wall 502c is 0.7 mm to 1.5 mm. As mentioned above, the thickness of the inclined wall 502c may be uniform or nonuniform. When the inclined wall 502c has the nonuniform thickness, the thickness of the inclined wall 502c refers to an average thickness of the inclined wall 502c, which is 0.7 mm to 1.5 mm.

In some other optional implementations, the inner aperture of the first exhaust hole 511 is greater than the outer aperture thereof; in these embodiments, the thickness of the inclined wall 502c is 0.7 mm to 2.0 mm. As mentioned above, the thickness of the inclined wall 502c may be uniform or nonuniform. When the inclined wall 502c has the nonuniform thickness, the thickness of the inclined wall 502c refers to an average thickness of the inclined wall 502c, which is 0.7 mm to 2.0 mm.

Preferably, the first exhaust hole 511 may be constructed as a tapered hole with a gradually increased area from the inner side to the outside of the tapered boss 502. The first exhaust hole 511 constructed in such a way is beneficial to a better divergency of the airflow, the airflow can be rapidly attenuated after flowing out of the first exhaust hole 511, and the impact energy of the airflow is lower, so that a bed partner of the patient cannot be disturbed. Moreover, mutual interference between the first exhaust holes 511 is not easy to occur, and thus, noise can be further lowered during use.

Optionally, the first exhaust hole 511 may also be constructed as a tapered hole with a gradually reduced area from the inner side to the outside of the tapered boss 502.

Further, when observed from the axial direction (Y direction) of the tapered boss 502, the first exhaust holes 511 are formed using molds, in particular, through the kiss off of an upper mold and a lower mold. Compared with a way of formation by a plurality of slides or later laser drilling during lateral opening in the related art, the way of forming the first exhaust hole 511 in present disclosure is better in technical moldability, lower in cost, capable of ensuring quality of the holes, and has less noise during use.

The first exhaust hole 511 may also in the shape of a strip, a long ellipse (as shown in FIG. 21), a circle or an ellipse. A radial cross section of the first exhaust hole 511 may also be configured as a special-shaped hole (such as a structure composed of at least two relatively parallel straight lines and at least one arc connected to the two straight lines).

The first exhaust holes 511 may be arranged in an array in the circumferential direction of the tapered boss 502, that is, the first exhaust holes 511 are arranged in the direction according to a certain rule. For example, the plurality of first exhaust holes 511 may be evenly distributed in a concentric circle concentric with the connecting aperture 52, or the plurality of first exhaust holes 511 are distributed in a certain ellipse. Alternatively, some of the plurality of first exhaust holes 511 are distributed in a certain ellipse, and other first exhaust holes 511 are distributed in another ellipse concentric with the ellipse.

With reference to implementations shown in FIG. 21, FIG. 24b, and FIG. 26, the main body of the exhaust assembly is of an elliptical structure. The first exhaust holes 511 are disposed to be close to an edge of the tapered boss 502 in a arrangement, and at most two neighboring first exhaust holes are located around each of the first exhaust holes 511. In other words, there is provided with merely one row of first exhaust holes 511 in a geometric extension direction of the tapered boss 502 (for example the axial direction of the tapered boss 502), and thus, each first exhaust hole 511 has at most two neighboring exhaust holes on the left side and the right side.

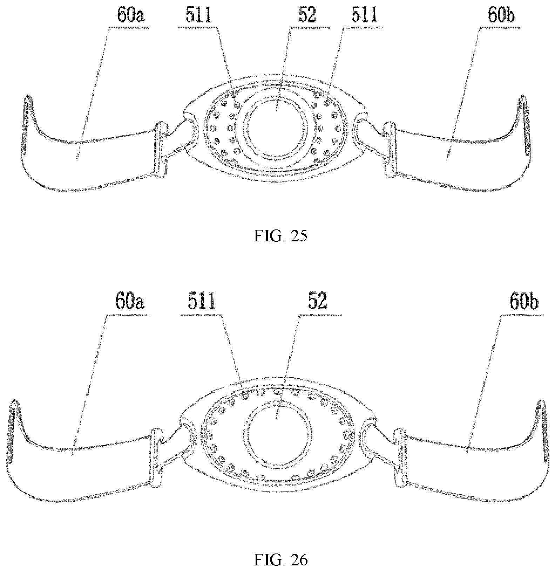

With reference to an implementation shown in FIG. 25, the first exhaust holes 511 may only be disposed on two sides of the connecting aperture 52, and three or more neighboring exhaust holes may be located around each first exhaust hole 511.

It can be understood that, when no elbow 90 is disposed on the frame 50, as mentioned in various implementations described above, the overall patient interface device 5 may be more compact and lighter, and dragging brought by a large tube connected to a pressure device can be overcome by means of the flexibility of a flexible hose 81. In these embodiments, the exhaust part 51 is the first exhaust holes 511 provided on the frame main body 501, or the exhaust part 51 is third exhaust holes 123 provided on the lower part of the nasal cushion 100, 200.

In various implementations described below, the exhaust part 51 (second exhaust holes 91) will be provided an elbow 90 rotatably connected to the frame main body 501.

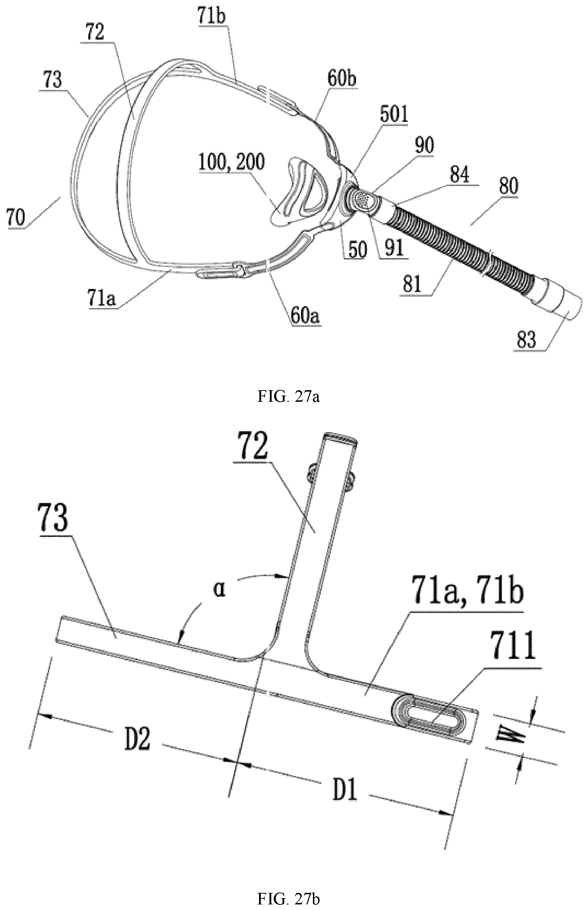

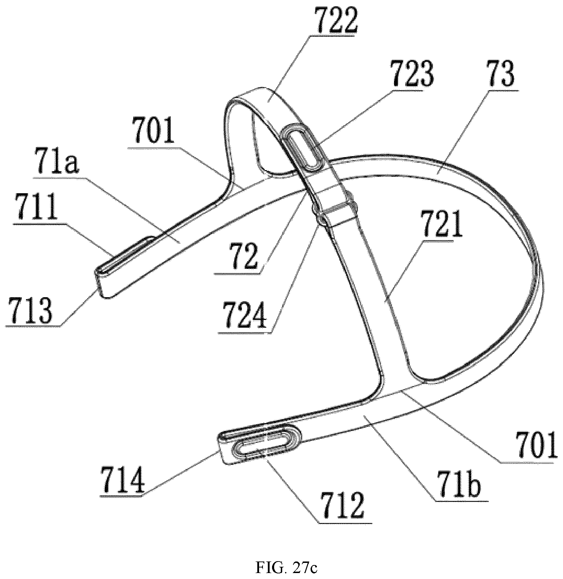

Specifically, as shown in FIG. 27, the frame 50 includes a frame main body 501 and an elbow 90 rotatably connected to the frame main body 501, a connecting aperture 52 passing through the thickness direction of the frame main body 501 is disposed at a central position of the frame main body 501. The elbow 90 is connected to the connecting aperture 52 and a vent tube 80, respectively. By disposing the elbow 90, a relative rotation between the vent tube 80 and the frame main body 501 may be realized, which facilitates adapting to various states of patients.

In an embodiment, the exhaust part 51 includes a second exhaust part provided on a side wall of the elbow 90. Moreover, the second exhaust part may also be configured as a plurality of second exhaust holes 91. Specifically, the side wall of the elbow 90 is provided with a recess that is recessed inward, and the second exhaust holes 91 are centrally disposed on the recess.

When the frame 50 is provided with the elbow 90, the elbow 90 may be rotatably connected to a second connecting end 82, so that it can be used more flexibly. In these embodiments, the exhaust part 51 is the second exhaust holes 91 provided on the elbow 90, or the exhaust part 51 is the third exhaust holes 123 provided at the lower part of the nasal cushion 100, 200.

In a further optional implementation, as shown in FIG. 21, the frame 50 includes a frame main body 501, a connecting aperture 52 passing through the thickness direction of the frame main body 501 is disposed at a central position of the frame main body 501, and a vent tube 80 is directly connected to the connecting aperture 52. In the present implementation, the exhaust part 51 may be disposed on the lower part 120, 220 of the nasal cushions 100, 200, as configured in embodiment 7; or the exhaust part 51 is configured as that in other embodiments described above.

The vent tube 80 is configured as a flexible hose 81 with an internal diameter of 15-18 mm, preferably 15 mm. Specifically, the flexible hose 81 includes a first connecting end 84 connected to the connecting aperture 52. The flexible hose 81 also includes a second connecting end 82 that may be made of a rigid material (for example, thermoplastics such as PP and PC). The second connecting end 82 is rotatably connected to a tube connector 83, and the tube connector 83 communicates with a pressure device by an air delivery tube. The air delivery tube may have the inner diameter of 15~22 mm and the length of 1000-2000 mm. Since the flexibility of the flexible hose 81 is better than that of the air delivery tube, it has the advantage of counteracting a dragging force generated by the air delivery tube during body movement, thereby keeping a mask system stably sealed on the face of a user.

Through the rotation between the second connecting end 82 and the tube connector 83, different postures of patients can be adapted. A head strap 70 in the present disclosure will be described in detail below. It can be understood that the head strap 70 described below may be combined with various preferred or optional frames 50 described above as well as any one of the nasal cushions 100, 200 in embodiments 1 to 6.

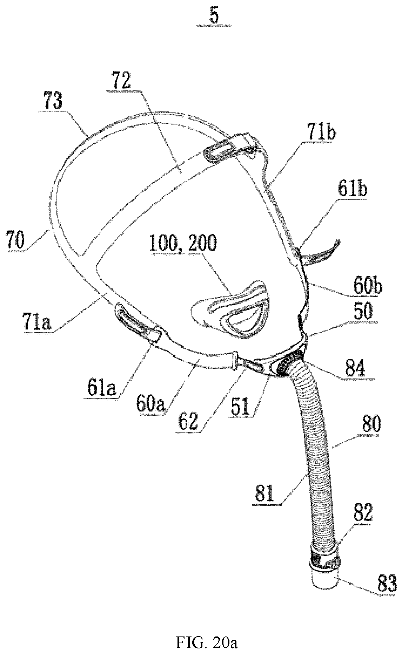

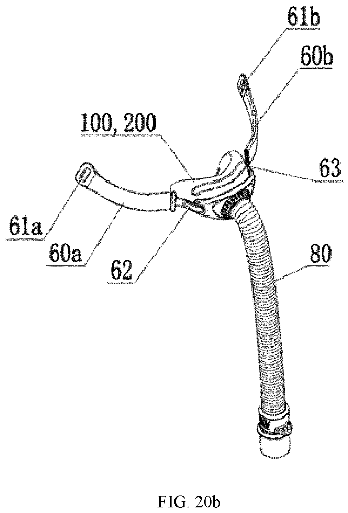

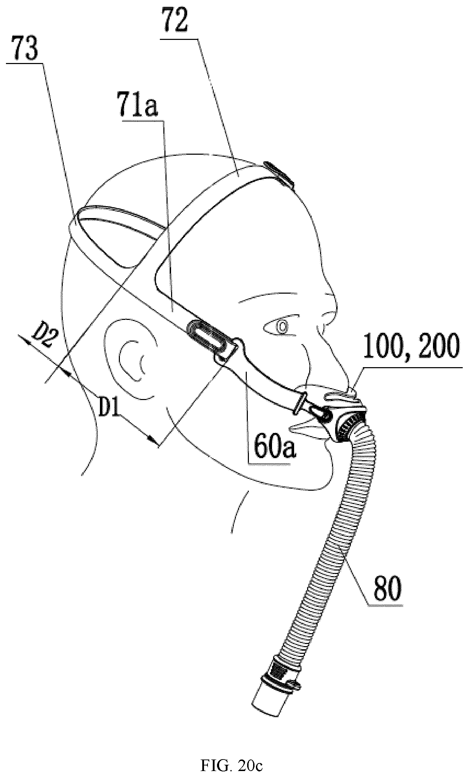

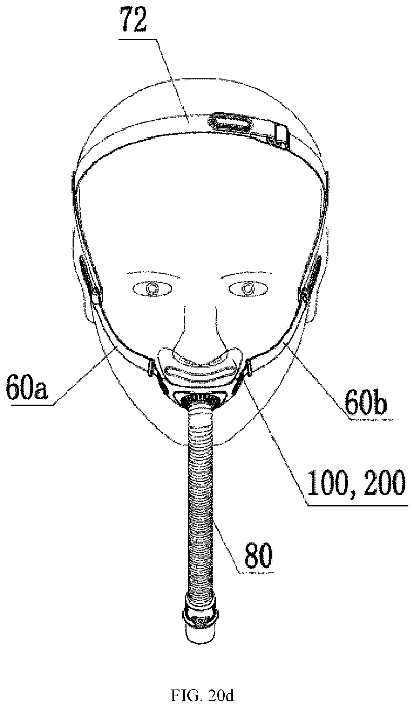

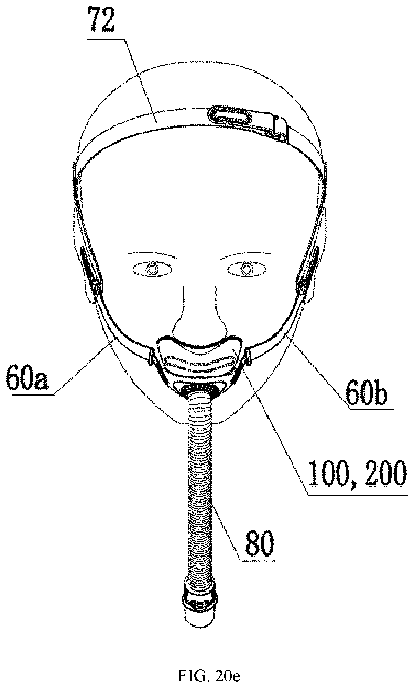

With reference to FIG. 20a, FIG. 20b, FIG. 20c, FIG. 20d, and FIG. 20e, the frame 500 includes a frame main body 501 as well as a first engaging lug 53 and a second engaging lug 54 that are disposed on two ends of the frame main body 501. The first engaging lug 53 is connected to a first skeleton arm 60a, and the second engaging lug 54 is connected to a second skeleton arm 60b. When being worn, the first engaging lug 53 extends along the cheeks of the patient from the left side of the frame main body 501 to the head strap 70 in a direction pointing to the upside of the left ear, and the second engaging lug 54 extends along the cheeks of the patient from the right side of the frame main body 501 to the head strap 70 in a direction pointing to the upside of the right ear.

It can be understood that the frame main body 501 may be formed by one-time integrated injection molding, i.e., integrated molding, with the first skeleton arm 60a and the second skeleton arm 60b by adopting the same material. Alternatively, the frame main body 501 may adopt materials different from the first skeleton arm 60a and the second skeleton arm 60b, and is connected to the first skeleton arm 60a and the second skeleton arm 60b in other ways, for example, through a chemical binder or in a mechanical way.

Preferably, the frame main body 501 is made of PC, and the first skeleton arm 60a and the second skeleton arm 60b are both made of PP. In addition, the frame main body 501 may also be made of other thermoplastic materials such as PP and ABS, and the first skeleton arm 60a and the second skeleton arm 60b may also be made of other thermoplastic materials such as PC and ABS.

The flexibility of the first skeleton arm 60a and the second skeleton arm 60b is better than the flexibility of the frame main body 501, and the thickness of a main part of the first skeleton arm 60a and the second skeleton arm 60b may be 0.6 to 1.8 mm, preferably 0.9 to 1.1 mm. By adopting the disposing way in the embodiment, the first skeleton arm 60a and the second skeleton arm 60b have better adaptability so as to be fitted to the cheeks of different patients to play a stable supporting role, and are configured to stably fix the nasal cushion 100, 200 to the face of the patient together with the head strap 70.

The head strap 70 includes a first side head strap 71a connected to the first skeleton arm 60a, a second side head strap 71b connected to the second skeleton arm 60b as well as an upper head strap 72 and a rear head strap 73 that are disposed between the first side head strap 71a and the second side head strap 71b. The upper head strap 72 and the rear head strap 73 are configured to be fixed respectively around the top and back of the head of the patient in response to the patient interface device 5 being worn by the patient.

When being worn, the first side head strap 71a extends backward from the first skeleton arm 60a above the left ear of the patient, and the second side head strap 71b extends backward from the second skeleton arm 60b above the right ear of the patient.

Specifically, the first skeleton arm 60a and the second skeleton arm 60b are respectively provided with a first reeving hole 61a and a second reeving hole 61b an end thereof, and the first side head strap 71a and the second side head strap 71b are respectively provided with a hook and loop tapeon an end thereof. The first side head strap 71a and the second side head strap 71b pass through the first reeving hole 61a and the second reeving hole 61b respectively, and are reversely folded to be attached or buckled to exteriors of the first side head strap 71a and the second side head strap 71b, so that the first side head strap 71a is connected to the first skeleton arm 60a, and the second side head strap 71b is connected to the second skeleton arm 60b. By adjusting a buckling or fitting position, wearing tensions of the first side head strap 71a and the second side head strap 71b can be adjusted to adapt to heads of different patients. For example, if the size of the head of a patient is large, the buckling or fitting position may be selected to be closer to the first skeleton arm 60a and the second skeleton arm 60b, and vice versa.

As shown in FIG. 27a, FIG. 27b, FIG. 27c, and FIG. 20c, extension directions of the first side head strap 71a and the second side head strap 71b are respectively located on the same straight line with an extension direction of a corresponding side of the rear head strap 73. Therefore, the adjustable range of the head strap 70 may be wider. Specifically, a first adjusting structure 711 is disposed on a first end of the first side head strap 71a, and a second adjusting structure 712 is disposed on a first end of the second side head strap 71b; and the first adjusting structure 711 and the second adjusting structure 712 are respectively configured to adjust use lengths of the first side head strap 71a and the second side head strap 71b so as to adjust a tightening force of the patient interface device.

With the first adjusting structure 711 and the second adjusting structure 712 being both Velcro as an example, as shown in FIG. 27b and FIG. 27c, after respectively passing through a corresponding connecting aperture 60 in the main part 100, the first adjusting structure 711 and the second adjusting structure 712 are reversely folded and attached to outer side surfaces of the first side head strap 71a and the second side head strap 71b. In this way, the first side head strap 71a forms a first reverse folding part 713, and the second side head strap 71b forms a second reverse folding part 714. Since the adjusting effects of the first side head strap 71a and the second side head strap 71b play a main role in wearing the patient interface device, the use lengths of the first side head strap 71a and the second side head strap 71b can be adjusted by reverse folding lengths of the first side head strap 71a and the second side head strap 71b, and then, the tightening force of the patient interface device is adjusted.

As mentioned above, since the extension directions of the first side head strap 71a and the second side head strap 71b are respectively approximately located on the same straight line with extension directions of two sides of the rear head strap 73, adjusting ranges of the first side head strap 71a and the second side head strap 71b are wider than an adjusting range D1 of a traditional easily-used head strap, that is, the adjusting ranges of the first side head strap 71a and the second side head strap 71b are D1+D2 (as shown in FIG. 27b and FIG. 20c). In other words, the rear head strap 73 also becomes a part to be adjusted. Therefore, the adjusting capacity of the head strap 70 is greatly greater than the adjusting capacity of a traditional Y-shaped head strap.

Further, a value range of D1 is 70~150 mm, preferably 85-120 mm. It can be understood that the adjusting range D1 is a distance from the first side head strap 71a to a joint between the upper head strap 72 and the rear head strap 73 described below, or a distance from the second side head strap 71b to a joint between the upper head strap 72 and the rear head strap 73 described below. In other words, the distance is measured in the extension direction of the first side head strap 71a or the second side head strap 71b.

It can be understood that the first adjusting structure 711 and the second adjusting structure 712 may be other connection structures such as a hasp, a magnetic attraction structure, a snap fastener and a buckle.

The width W of the first side head strap 71a is 12~16 mm; and the width W of the second side head strap 71b is 12~16 mm. It should be noted that the "width" refers to a size in a direction perpendicular to the extension direction of the first side head strap 71a or the second side head strap 71b, as shown in FIG. 27b. A width that is too wide or too narrow will cause wearing discomfort. For example, if the width is too narrow, local strangulation marks on the face and head of a user will be caused, and if the width is too wide, excess skin of the user will be covered. Therefore, the first side head strap 71a and the second side head strap 71b within the above-mentioned width range can improve the wearing comfort of the user on the premise of ensuring that the patient interface device is closely fixed to the face of the user.

Optionally, the rear head strap 73 is provided with a third adjusting structure for adjusting a use length of the rear head strap 73. The third adjusting structure may be similar to the first adjusting structure 711 and the second adjusting structure 712, and is other connection structures such as a hasp, a magnetic attraction structure, a snap fastener and a buckle, thereby further adjusting the use length of the head strap 70.

The length of the rear head strap 73 is 200-260 mm, and the rear head strap 73 within such length range can satisfy requirements of head circumferences of most of people.

The head strap 70 in the present disclosure also includes the upper head strap 72, and two ends of the upper head strap 72 are respectively connected to two sides of the rear head strap 73, as shown in FIG. 27b; and an included angle $\alpha$ between the upper head strap 72 and the rear head strap 73 is 30° to 150°, preferably 75° to 85°, so that the rear head strap 73 and the upper head strap 72 can be firmly combined to the head of the user.

As shown in an optional embodiment in FIG. 27c and FIG. 20c, the upper head strap 72 includes a first end strap 721 and a second end strap 722. The first end strap 721 extends from a joint between the second end of the first side head strap 71a and one side of the rear head strap 73 to the second end strap 722, and the second end strap 722 extends from a joint between the second end of the second side head strap 71b and the other side of the rear head strap 73 to the first end strap 721. The first end strap 721 is connected to the second end strap 722 by a fourth adjusting structure 723. Since the upper head strap 72 can also play an auxiliary adjusting role, the fourth adjusting structure 723 is disposed to adjust the use length of the upper head strap 72.

The fourth adjusting structure 723 includes a connecting member disposed on one of the first end strap 721 and the second end strap 722 and a matching member disposed on the other one of the both, and the matching member is matched and connected to the connecting member.

For example, the connecting member and the matching member may be other connection structures such as a Velcro, a hasp, a magnetic attraction structure, a snap fastener and a buckle.

In an embodiment, the connecting member is a head strap buckle 724 disposed on the first end strap 721, the matching member is a Velcro disposed on the second end strap 722, the Velcro is reversely folded and attached to the outer side surface of the second end strap 722 after passing through the head strap buckle 724, and an actual tightening force of the upper head strap 72 can be adjusted by means of a reverse folding length. The fourth adjusting structure 723 is the same as the above-mentioned adjusting structures, and can adjust the use length of the upper head strap 72 to be adapted to users with different head circumferences.

Further, in the embodiment provided with the fourth adjusting structure 723, the length of the upper head strap 72 is 190-310 mm, preferably 230-270 mm. The length of the upper head strap 72 is an unfolding length between the joints of two ends of the upper head strap 72 and the rear head strap 73 when the Velcro on the second end strap 722 passes through the head strap buckle 724, and is cohered after being folded back on a position closest to the root so that the first end strap 721 is connected to the second end strap 722 (or it can be understood as an arc length between the two ends of the upper head strap 72).

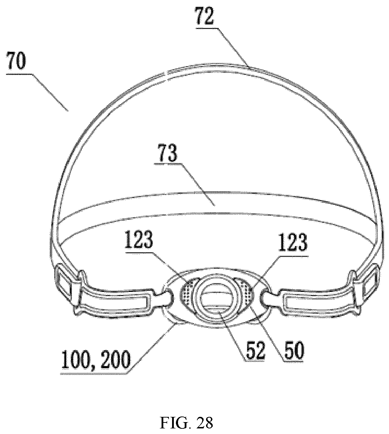

As shown in FIG. 28, in another optional embodiment, the upper head strap 72 is not provided with the adjusting structure, which can reduce the cost.

Further, in the embodiment without the adjusting structure, the length of the upper head strap 72 is 190-310 mm, preferably 230-270 mm. It can be understood that, in the embodiment, the length of the upper head strap 72 is an unfolding length from the joints between two ends thereof and the rear head strap 73 (or it can be understood as an arc length between the two ends of the upper head strap 72).

In addition, in the above-mentioned preferred or optional embodiment, for facilitating adjusting, the length of the first end strap 721 may be set to be unequal to the length of the second end strap 722. As shown in FIG. 27c, the length of the first end strap 721 provided with the Velcro may be set to be greater than the length of the second end strap 722. In some preferred implementations, the rear head strap 73, the first side head strap 71a and the second side head strap 71b are formed by integrally cutting the same fabric, and are then spliced with the upper head strap 72 at a splicing line 701 shown in FIG. 27c.

In some optional implementations, the upper head strap 72, the rear head strap 73, the first side head strap 71a and the second side head strap 71b are formed by integrally cutting the same fabric.

In some optional implementations, the first side head strap 71a and the second side head strap 71b as well as the rear head strap 73 and the upper head strap 72 can also be formed by splicing three pieces of fabrics.

In addition, the first side head strap 71a, the second side head strap 71b, the upper head strap 72 and the rear head strap 73 can be all made of foam, silicon rubber, elastic fabrics or breathable fabrics.

The head strap 70 is of an integrated structure, that is, the first side head strap 71a, the second side head strap 71b, the upper head strap 72 and the rear head strap 73 are integrated with each other. Therefore, the head strap 70 can be configured to have a smaller width than an existing four-point head strap. For example, the width of the head strap 70 may be 8~16 mm, so that the better flexibility is achieved. In addition, the head strap 70 does not pass through downsides of the ears of the patient when being worn, in this way, the head strap 70 does not need to be taken off when it is worn by a patient, and therefore, it is very convenient and easy to use; and the head strap 70 can be conveniently worn and taken off by only adjusting the wearing tension of the head strap 70 one time, and the wearing tension of the head strap 70 does not need to be repeatedly adjusted when the head strap 70 is worn or taken off every time.

The invention is defined in independent claim 1. Preferred embodiments are matter of the appended dependent claims.

## Claims

1. An exhaust assembly disposed on a patient interface device, comprising a main body, wherein the main body comprises:

   a tapered boss (502), wherein the tapered boss (502) is provided with first exhaust holes (511) arranged in an array in a circumferential direction of the tapered boss (502), and the first exhaust holes (511) are formed through kiss off of an upper mold and a lower mold; wherein the tapered boss (502) comprises an inner wall (502a), an outer wall (502b), and an inclined wall (502c) disposed between the inner wall (502a) and the outer wall (502b), and the first exhaust holes (511) are disposed on the inclined wall (502c).

2. The exhaust assembly according to claim 1, wherein an inner aperture of each of the first exhaust holes (511) is greater than an outer aperture of each of the first exhaust holes (511), and a thickness of the inclined wall (502c) is 0.7mm to 2.0mm, or wherein an inner aperture of each of the first exhaust holes (511) is smaller than an outer aperture of each of the first exhaust holes (511), and a thickness of the inclined wall (502c) is 0.7mm to 1.5mm.

3. The exhaust assembly according to claim 1 or 2, wherein each of the first exhaust holes (511) is in a shape of a long ellipse, a rectangle or a structure composed of at least two relatively parallel straight lines and at least one arc connected to the two straight lines.

4. The exhaust assembly according to claim 1, wherein the first exhaust holes (511) are round holes or taper holes.

5. A frame assembly used for a patient interface device, wherein the frame main body (501) is provided with the exhaust assembly according to any one of claims 1 to 4, comprising:

   a frame main body (501) with one side being connected to an intake tube, and the other side being connected to a cushion assembly; a pair of skeleton arms comprising a first skeleton arm (60a) and a second skeleton arm (60b) disposed oppositely on the frame main body (501); and a connecting body (55) configured to connect the cushion assembly to the frame main body (501), wherein the connecting body (55) and the cushion assembly are sealed to form a closed connection, the connecting body (55) is provided with a boss raised from an inner side of the frame main body (501), a recess (56) for accommodating the cushion assembly is formed between the boss and an inner wall (502a) of the frame main body (501), and the boss extends into a bottom of the cushion assembly.

6. The frame assembly according to claim 5, wherein the exhaust assembly and the frame main body (501) are integrally molded, or the skeleton arms (60a, 60b) and the frame main body (501) are integrally molded.

7. A patient interface device, comprising: a cushion assembly comprising a nasal cushion (100, 200) configured to be in contact with a face of a patient, wherein the nasal cushion (100, 200) is configured to be adaptively fitted around nostrils of the patient in response to the patient interface device being worn by the patient, and the nasal cushion (100, 200) is configured to, when being pressed against the basis nasi of the patient due to a pressurized gas to seal, apply no clamping effect on the ala nasi of the patient;

   the frame assembly according to claim 5 or 6 with two sides being connected to the nasal cushion (100, 200) and a vent tube (80), respectively; and a head strap (70) connected to the frame, wherein the head strap (70) is configured to be fixed around the head of the patient in response to the patient interface device being worn by the patient wherein the cushion assembly comprises an upper part (110, 120), a lower part (120, 220) opposite to the upper part (110, 120), and a circumferential side part disposed between the upper part (110, 120) and the lower part (120, 220), and the upper part (110, 120) and the lower part (120, 220) surrounded by the circumferential side part to form a cavity (150, 250); the upper part (110, 120) comprises a face contact part (111a, 211a) to be in contact with a face of a user;

wherein the face contact part (111a, 211a) comprises a middle part (111, 211) as well as a first side part (112, 212) and a second side part (113, 213) located on two sides of the middle part (111, 211), and the first side part (112, 212) and the second side part (113, 213) are both connected to the circumferential side part; and the middle part (111, 211) comprises a nose opening (1111, 2111) communicated with the cavity (150, 250) and a periphery sealing part (1112, 2112) surrounding the nose opening (1111, 2111), and the nose opening (1111, 2111) is configured to surround lower sides of nostrils of a user in response to the nasal cushion (100, 200) being worn by the user, and is adaptively fitted around the nostrils of the user through the periphery sealing part (1112, 2112) to seal.

8. The patient interface device according to claim 7, wherein the frame assembly comprises the frame main body (501) and an elbow (90) rotatably connected to the frame main body (501), and the elbow (90) is connected to the vent tube (80) in a sealing way, or
the frame assembly is directly connected to an elbow (90), the elbow (90) is connected to an air delivery tube configured to be connected to a pressure device; and an inner diameter of the air delivery tube is 15 mm to 22 mm.

9. The patient interface device according to claim 7, wherein the frame assembly is directly connected to a flexible hose (81), the flexible (81) is connected to an air delivery tube configured to be connected to a pressure device; and an inner diameter of the flexible hose (81) is 12 mm to 15 mm, and the inner diameter of the air delivery tube is 15 mm to 22 mm;
wherein the flexible hose (81) is an elastic tube.

10. The patient interface device according to claim 7, wherein a connecting body (55) is provided on an inner side of the frame, and the nasal cushion (100, 200) is connected to the frame in a sealing way through the connecting body (55);
wherein a base (121, 221) is provided on a lower side of the nasal cushion (100, 200), and a bottom opening (122, 222) is provided on the base (121, 221); and the connecting body (55) is configured as a boss raised from the inner side of the frame, a recess (56) is formed between the boss and an inner wall (502a) of the frame, the base (121, 221) of the nasal cushion (100, 200) is disposed in the recess, and the boss extends into the bottom opening (122, 222).

11. The patient interface device according to claim 7, further comprising a first skeleton arm (60a) and a second skeleton arm (60b) respectively disposed on two ends of the frame, wherein the first skeleton arm (60a) and the second skeleton arm (60b) extend to upsides of ears along cheeks of the wearer respectively in response to the patient interface device being worn;
wherein the head strap (70) comprises a first side head strap (71a) connected to the first skeleton arm (60a), a second side head strap (71b) connected to the second skeleton arm (60b) as well as an upper head strap (72) and a rear head strap (73) that are disposed between the first side head strap (71a) and the second side head strap (71b); the upper head strap (72) and the rear head strap (73) are configured to be fixed respectively 15 around a back and top of the head of the patient in response to the patient interface device being worn by the patient.

**Patentansprüche**

1. Auslassanordnung, die an einer Patientenschnittstellenvorrichtung angeordnet ist und einen Hauptkörper aufweist, wobei der Hauptkörper aufweist:

einen konischen Vorsprung (502), wobei der konische Vorsprung (502) mit ersten Auslassöffnungen (511) versehen ist, die in einer Anordnung in Umfangsrichtung des konischen Vorsprungs (502) angeordnet sind, und die ersten Auslassöffnungen (511) durch Abtrennen einer oberen Form und einer unteren Form gebildet sind;
wobei der konische Vorsprung (502) eine Innenwand (502a), eine Außenwand (502b) und eine zwischen der Innenwand (502a) und der Außenwand (502b) angeordnete, geneigte Wand (502c) aufweist und die ersten Auslassöffnungen (511) an der geneigten Wand (502c) angeordnet sind.

2. Auslassanordnung gemäß Anspruch 1, wobei eine innere Öffnung jedes der ersten Auslassöffnungen (511) größer ist als eine äußere Öffnung jedes der ersten Auslassöffnungen (511), und eine Dicke der geneigten Wand (5020) 0,7 mm bis 2,0 mm beträgt, oder
wobei eine innere Öffnung jeder der ersten Auslassöffnungen (511) kleiner ist als eine äußere Öffnung jeder der ersten Auslassöffnungen (511) und eine Dicke der geneigten Wand (502c) 0,7 mm bis 1,5 mm beträgt.

3. Auslassanordnung gemäß Anspruch 1 oder 2, wobei jede der ersten Auslassöffnungen (511) die Form einer langen Ellipse, eines Rechtecks oder einer Struktur aufweist, die aus mindestens zwei relativ parallelen Geraden und mindestens einem Bogen besteht, der mit den beiden Geraden verbunden ist.

**4.** Auslassanordnung gemäß Anspruch 1, wobei die ersten Auslassöffnungen (511) runde Öffnungen oder konische Öffnungen sind.

**5.** Rahmenanordnung für eine Patientenschnittstellenvorrichtung, wobei ein Rahmenhauptkörper (501) mit der Auslassanordnung gemäß einem der Ansprüche 1 bis 4 versehen ist, mit:

dem Rahmenhauptkörper (501), dessen eine Seite mit einem Einlassrohr verbunden ist und dessen andere Seite mit einer Polsteranordnung verbunden ist;
zwei als Paar vorgesehene Gerüstarme mit einem ersten Gerüstarm (60a) und einem zweiten Gerüstarm (60b), die gegenüberliegend an dem Rahmenhauptkörper (501) angeordnet sind; und
einem Verbindungskörper (55), der ausgebildet ist, die Polsteranordnung mit dem Rahmenhauptkörper (501) zu verbinden, wobei der Verbindungskörper (55) und die Polsteranordnung abgedichtet sind, um eine geschlossene Verbindung zu bilden, der Verbindungskörper (55) mit einem Vorsprung versehen ist, der von einer Innenseite des Rahmenhauptkörpers (501) absteht, zwischen dem Vorsprung und einer Innenwand (502a) des Rahmenhauptkörpers (501) eine Aussparung (56) zur Aufnahme der Polsteranordnung ausgebildet ist und sich der Vorsprung in einen Boden der Polsteranordnung erstreckt.

**6.** Rahmenanordnung nach Anspruch 5, wobei die Auslassanordnung und der Rahmenhauptkörper (501) als Einheit geformt sind oder die Gerüstarme (60a, 60b) und der Rahmenhauptkörper (501) als Einheit geformt sind.

**7.** Patientenschnittstellenvorrichtung, mit:

einer Polsteranordnung mit einem Nasenkissen (100, 200), das ausgebildet ist, mit dem Gesicht eines Patienten in Kontakt zu stehen, wobei das Nasenkissen (100, 200) ausgelegt ist, sich in Reaktion darauf, dass die Patientenschnittstellenvorrichtung vom Patienten getragen wird, anpassungsfähig um die Nasenlöcher des Patienten zu legen, und das Nasenkissen (100, 200) ausgelegt ist, wenn es aufgrund eines unter Druck stehenden Gases zum Abdichten gegen die Nasenbasis des Patienten gedrückt wird, keine Klemmwirkung auf die Nasenflügel des Patienten auszuüben;
die Rahmenanordnung gemäß Anspruch 5 oder 6 mit zwei Seiten, die jeweils mit dem Nasenkissen (100, 200) und einer Entlüftungsleitung (80) verbunden sind; und

einem Kopfband (70), das mit dem Rahmen verbunden ist, wobei das Kopfband (70) ausgebildet ist, um den Kopf des Patienten befestigt zu werden, wenn die Patientenschnittstellenvorrichtung vom Patienten getragen wird, wobei die Polsteranordnung einen oberen Teil (110, 120), einen unteren Teil (120, 220) gegenüber dem oberen Teil (110, 120) und einen umlaufenden Seitenteil, der zwischen dem oberen Teil (110, 120) und dem unteren Teil (120, 220) angeordnet ist, aufweist, und wobei der obere Teil (110, 120) und der untere Teil (120, 220) von dem umlaufenden Seitenteil derart umgeben sind, dass sie einen Hohlraum (150, 250) bilden;
wobei der obere Teil (110, 120) einen Gesichtskontaktteil (111a, 211a) aufweist, der mit dem Gesicht eines Benutzers in Kontakt kommt;
wobei der Gesichtskontaktteil (111a, 211a) einen Mittelteil (111, 211) sowie einen ersten Seitenteil (112, 212) und einen zweiten Seitenteil (113, 213) aufweist, die sich auf zwei Seiten des Mittelteils (111, 211) befinden, und der erste Seitenteil (112, 212) und der zweite Seitenteil (113, 213) jeweils mit dem umlaufenden Seitenteil verbunden sind; und der Mittelteil (111, 211) eine Nasenöffnung (1111, 2111), die mit dem Hohlraum (150, 250) in Verbindung steht, und ein die Nasenöffnung (1111, 2111) umgebendes peripheres Dichtungsteil (1112, 2112) aufweist, und die Nasenöffnung (1111, 2111) ausgebildet ist, die Unterseiten der Nasenlöcher eines Benutzers zu umschließen, wenn das Nasenkissen (100, 200) vom Benutzer getragen wird, und durch das periphere Dichtungsteil (1112, 2112) an die Nasenlöcher des Benutzers zum Abdichten anzupassen.

**8.** Patientenschnittstellenvorrichtung gemäß Anspruch 7, wobei die Rahmenanordnung den Rahmenhauptkörper (501) und ein Winkelstück (90) aufweist, das drehbar mit dem Rahmenhauptkörper (501) verbunden ist, und das Winkelstück (90) dichtend mit der Entlüftungsleitung (80) verbunden ist, oder
die Rahmenanordnung direkt mit einem Winkelstück (90) verbunden ist, das Winkelstück (90) mit einem Luftzufuhrschlauch verbunden ist, der zum Anschluss an eine Druckvorrichtung ausgelegt ist, und der Innendurchmesser des Luftzufuhrschlauchs 15 mm bis 22 mm beträgt.

**9.** Patientenschnittstellenvorrichtung gemäß Anspruch 7, wobei die Rahmenanordnung direkt mit einem flexiblen Schlauch (81) verbunden ist, der flexible Schlauch (81) mit einem Luftzufuhrschlauch verbunden ist, der ausgebildet ist, mit einer Druckvorrichtung verbunden zu werden; und ein Innendurchmesser des flexiblen Schlauchs (81) 12 mm bis 15 mm beträgt und der Innendurchmesser des Luftzufuhrschlauchs 15 mm bis 22 mm beträgt; wobei der flexible Schlauch (81) ein elastischer Schlauch ist.

10. Patientenschnittstellenvorrichtung gemäß Anspruch 7, wobei ein Verbindungskörper (55) auf einer Innenseite des Rahmens vorgesehen ist und das Nasenkissen (100, 200) mit dem Rahmen durch den Verbindungskörper (55) abdichtend verbunden ist; wobei eine Basis (121, 221) an einer Unterseite des Nasenkissens (100, 200) vorgesehen ist und eine Bodenöffnung (122, 222) an der Basis (121, 221) vorgesehen ist; und der Verbindungskörper (55) als ein Vorsprung ausgebildet ist, der von der Innenseite des Rahmens absteht, zwischen dem Vorsprung und einer Innenwand (502a) des Rahmens eine Aussparung (56) ausgebildet ist, die Basis (121, 221) des Nasenkissens (100, 200) in der Aussparung angeordnet ist und sich der Vorsprung in die Bodenöffnung (122, 222) erstreckt.

11. Patientenschnittstellenvorrichtung gemäß Anspruch 7, die ferner einen ersten Gerüstarm (60a) und einen zweiten Gerüstarm (60b) aufweist, die entsprechend an zwei Enden des Rahmens angeordnet sind, wobei sich der erste Gerüstarm (60a) und der zweite Gerüstarm (60b) entsprechend dem Tragen der Patientenschnittstellenvorrichtung entsprechend entlang der Wangen des Trägers zu den Ohren erstrecken; wobei das Kopfband (70) ein erstes seitliches Kopfband (71a), das mit dem ersten Gerüstarm (60a) verbunden ist, ein zweites seitliches Kopfband (71b), das mit dem zweiten Gerüstarm (60b) verbunden ist, sowie ein oberes Kopfband (72) und ein hinteres Kopfband (73), die zwischen dem ersten seitlichen Kopfband (71a) und dem zweiten seitlichen Kopfband (71b) angeordnet sind, aufweist; wobei das obere Kopfband (72) und das hintere Kopfband (73) ausgebildet sind, entsprechend um den Hinterkopf und die Kopfoberseite des Patienten herum befestigt zu werden, wenn die Patientenschnittstellenvorrichtung vom Patienten getragen wird.

**Revendications**

1. Ensemble d'expiration disposé sur un dispositif d'interface patient, comprenant un corps principal, dans lequel le corps principal comprend :

   une bosse effilée (502), dans lequel la bosse effilée (502) est pourvue de premiers trous d'expiration (511) agencés en une rangée dans une direction circonférentielle de la bosse effilée (502), et les premiers trous d'expiration (511) étant formés à travers un décollement d'un moule supérieur et d'un moule inférieur ; dans lequel la bosse effilée (502) comprend une paroi interne (502a), une paroi externe (502b), et une paroi inclinée (502c) disposée entre la paroi interne (502a) et la paroi externe (502b), et les premiers trous d'expiration (511) sont disposés sur la paroi inclinée (502c).

2. Ensemble d'expiration selon la revendication 1, dans lequel une ouverture interne de chacun des premiers trous d'expiration (511) est supérieure à une ouverture externe de chacun des premiers trous d'expiration (511), et une épaisseur de la paroi inclinée (502c) est de 0,7 mm à 2,0 mm, ou dans lequel une ouverture interne de chacun des premiers trous d'expiration (511) est inférieure à une ouverture externe de chacun des premiers trous d'expiration (511), et une épaisseur de la paroi inclinée (502c) est de 0,7 mm à 1,5 mm.

3. Ensemble d'expiration selon la revendication 1 ou 2, dans lequel chacun des premiers trous d'expiration (511) se présente sous une forme d'une longue ellipse, d'un rectangle ou d'une structure composée d'au moins deux lignes droites relativement parallèles et d'au moins un arc raccordé aux deux lignes droites.

4. Ensemble d'expiration selon la revendication 1, dans lequel les premiers trous d'expiration (511) sont des trous ronds ou des trous effilés.

5. Ensemble formant cadre utilisé pour un dispositif d'interface patient, dans lequel le corps principal de cadre (501) est pourvu de l'ensemble d'expiration selon l'une quelconque des revendications 1 à 4, comprenant :

   un corps principal de cadre (501) ayant un côté raccordé à un tube d'entrée, et l'autre côté étant raccordé à un ensemble coussinet ; une paire de bras de squelette comprenant un premier bras de squelette (60a) et un second bras de squelette (60b) disposé à l'opposé sur le corps principal de cadre (501) ; et un corps de raccordement (55) configuré pour raccorder l'ensemble coussinet au corps principal de cadre (501), dans lequel le corps de raccordement (55) et l'ensemble coussinet sont scellés pour former un raccordement fermé, le corps de raccordement (55) est pourvu d'une bosse élevée depuis un côté interne du corps principal de cadre (501), un renfoncement (56) pour loger l'ensemble coussinet est formé entre la bosse et une paroi interne (502a) du corps principal de cadre (501), et la bosse s'étend dans un fond de l'ensemble coussinet.

**6.** Ensemble formant cadre selon la revendication 5, dans lequel l'ensemble d'expiration et le corps principal de cadre (501) sont intégralement moulés, ou les bras de squelette (60a, 60b) et le corps principal de cadre (501) sont intégralement moulés.

**7.** Dispositif d'interface patient, comprenant : un ensemble coussinet comprenant un coussinet nasal (100, 200) configuré pour se trouver en contact avec un visage d'un patient, dans lequel le coussinet nasal (100, 200) est configuré pour être ajusté de manière adaptative autour des narines du patient en réponse au dispositif d'interface patient étant porté par le patient, et le coussinet nasal (100, 200) est configuré pour, lorsqu'il est pressé contre la base du nez du patient dû à un gaz sous pression pour l'étanchéité, n'appliquer aucun effet de serrage sur les ailes du nez du patient ;

l'ensemble formant cadre selon la revendication 5 ou 6 avec deux côtés étant raccordé au coussinet nasal (100, 200) et un tube de ventilation (80), respectivement ; et une sangle pour la tête (70) raccordée au cadre, dans lequel la sangle pour la tête (70) est configurée pour être fixée autour de la tête du patient en réponse au dispositif d'interface patient qui est porté par le patient dans lequel l'ensemble coussinet comprend une partie supérieure (110, 120), une partie inférieure (120, 220) opposée à la partie supérieure (110, 120), et une partie latérale circonférentielle disposée entre la partie supérieure (110, 120) et la partie inférieure (120, 220), et la partie supérieure (110, 120) et la partie inférieure (120, 220) entourées par la partie latérale circonférentielle pour former une cavité (150, 250) ; la partie supérieure (110, 120) comprend une partie de contact avec le visage (111a, 211a) pour se trouver en contact avec un visage d'un utilisateur ; dans lequel la partie de contact avec le visage (111a, 211a) comprend une partie médiane (111, 211) ainsi qu'une première partie latérale (112, 212) et une seconde partie latérale (113, 213) localisées sur deux côtés de la partie médiane (111, 211), et la première partie latérale (112, 212) et la seconde partie latérale (113, 213) sont toutes deux raccordées à la partie latérale circonférentielle ; et la partie médiane (111, 211) comprend une ouverture pour le nez (1111, 2111) en communication avec la cavité (150, 250) et une partie d'étanchéité périphérique (1112, 2112) entourant l'ouverture pour le nez (1111, 2111), et l'ouverture pour le nez (1111, 2111) est configurée pour entourer des côtés inférieurs des narines d'un utilisateur en réponse au coussinet nasal (100, 200) qui est porté par l'utilisateur, et est ajustée de manière adaptative autour des narines de l'utilisateur à travers la partie d'étanchéité périphérique (1112, 2112) pour une fermeture étanche.

**8.** Dispositif d'interface patient selon la revendication 7, dans lequel l'ensemble formant cadre comprend le corps principal de cadre (501) et un coude (90) raccordé de manière à pouvoir tourner au corps principal de cadre (501), et le coude (90) est raccordé au tube de ventilation (80) d'une manière étanche, ou l'ensemble formant cadre est directement raccordé à un coude (90), le coude (90) est raccordé à un tube de distribution d'air configuré pour être raccordé à un dispositif de pression ; et un diamètre interne du tube de distribution d'air est de 15 mm à 22 mm.

**9.** Dispositif d'interface patient selon la revendication 7, dans lequel l'ensemble formant cadre est directement raccordé à un tuyau flexible (81), le flexible (81) est raccordé à un tube de distribution d'air configuré pour être raccordé à un dispositif de pression ; et un diamètre interne du tuyau flexible (81) est de 12 mm à 15 mm, et le diamètre interne du tube de distribution d'air est de 15 mm à 22 mm ; dans lequel le tuyau flexible (81) est un tube élastique.

**10.** Dispositif d'interface patient selon la revendication 7, dans lequel un corps de raccordement (55) est prévu sur un côté interne du cadre, et le coussinet nasal (100, 200) est raccordé au cadre d'une manière étanche à travers le corps de raccordement (55) ; dans lequel une base (121, 221) est fournie sur un côté inférieur du coussinet nasal (100, 200), et une ouverture inférieure (122, 222) est fournie sur la base (121, 221) ; et le corps de raccordement (55) est configuré sous la forme d'une bosse élevée depuis le côté interne du cadre, un renfoncement (56) est formé entre la bosse et une paroi interne (502a) du cadre, la base (121, 221) du coussinet nasal (100, 200) est disposée dans le renfoncement, et la bosse s'étend dans l'ouverture inférieure (122, 222).

**11.** Dispositif d'interface patient selon la revendication 7, comprenant en outre un premier bras de squelette (60a) et un second bras de squelette (60b) respectivement disposés sur deux extrémités du cadre, dans lequel le premier bras de squelette (60a) et le second bras de squelette (60b) s'étendent vers les côtés supérieurs des oreilles le long des joues du porteur respectivement en réponse au dispositif d'interface patient étant porté ; dans lequel la sangle pour la tête (70) comprend une première sangle latérale pour la tête (71a) raccordée au premier bras de squelette (60a), une seconde sangle latérale pour la tête (71b) raccordée au se-

**EP 4 400 139 B1**

cond bras de squelette (60b) ainsi qu'une sangle supérieure pour la tête (72) et une sangle arrière pour la tête (73) qui sont disposées entre la première sangle latérale pour la tête (71a) et la seconde sangle latérale pour la tête (71b) ; la sangle supérieure pour la tête (72) et la sangle arrière pour la tête (73) sont configurées pour être respectivement fixées autour d'une partie arrière et d'un sommet de la tête du patient en réponse au dispositif d'interface patient étant porté par le patient.

FIG. 1

FIG. 2

FIG. 3

FIG. 4

FIG. 5

FIG. 6

FIG. 7

FIG. 8

FIG. 9

141

143

142

FIG. 10

110

143

131

142

120

FIG. 11

FIG. 12

FIG. 13

FIG. 14

FIG. 15

FIG. 16

FIG. 17

111a, 211a

51 (124)

51 (124)

122, 222

121, 221

FIG. 18

nasal
bridge

nasal
dorsum

apex nasi

ala nasi

nostril

nasal depression

nasal septum

nasal depression

FIG. 19

FIG. 20a

FIG. 20b

FIG. 20c

FIG. 20d

FIG. 20e

FIG. 21

viewed from X direction

FIG. 22

511   52

Y
Z
X

viewed from Y direction

FIG. 23

61b   60b   55   60a   61a

FIG. 24a

FIG. 24b

FIG. 24c

FIG. 25

FIG. 26

FIG. 27a

FIG. 27b

FIG. 27c

FIG. 28

FIG. 29

FIG. 30

**EP 4 400 139 B1**

**REFERENCES CITED IN THE DESCRIPTION**

**Patent documents cited in the description**

- US 20190030272 A1
- WO 2021046605 A1
- US 20150352306 A1
- US 20210244905 A1
- WO 2020208523 A1
- US 10912909 B2
- WO 2020136070 A1